# EUROPEAN PATENT SPECIFICATION

(11) **EP 3 071 970 B1**
(45) Date of publication and mention of the grant of the patent: **29.08.2018**
(21) Application number: 13802551.5
(22) Date of filing: 19.11.2013
(51) Int. Cl.: G01N 33/543

(54) **KIT, SOLID SUPPORT UNIT STRIP, HOLDER, AND METHOD FOR DETECTING AT LEAST TWO ANALYTES**
KIT, FESTTRÄGEREINHEITSSTREIFEN, HALTER UND VERFAHREN ZUR DETEKTION VON MINDESTENS ZWEI ANALYTEN
KIT, BANDELETTE D'UNITÉS DE SUPPORT SOLIDES, SUPPORT, ET PROCÉDÉ DE DÉTECTION D'AU MOINS DEUX ANALYTES

(43) Date of publication of application: 28.09.2016
(73) Proprietor: Tekinvest SPRL, 7000 Mons (BE)
(72) Inventor: VIGNERON, Alain Georges André, 7022 Hyon (BE); BODART, Nicolas, 7000 Mons (BE); GOOSSENS, Hans Achiel Tony, 8930 Menen (BE); ROYEN, François Gabriel Théo, 4357 Donceel (BE); AUTEM, Benoît, Valère, Jacques, Ghislain, 7000 Mons (BE)
(74) Representative: Gyi, Jeffrey Ivan
(86) International application number: PCT/EP2013/074190
(87) International publication number: WO 2015/074681

(56) References cited:
- WO-A1-2004/113901
- WO-A1-2010/022913
- WO-A1-2012/066032
- US-A1- 2005 100 880
- US-A1- 2008 166 820

## Description

### FIELD OF THE INVENTION

The invention is broadly in the field of analysis for example in the field of medical laboratory diagnostics. The present invention relates to a kit, a solid support unit strip, a holder, and uses thereof, and to methods for preparing a test strip for detecting at least two analytes in a biological sample. The invention allows the consumer to prepare a custom-made multi-parametric test strip for detecting at least two analytes in a biological sample.

### BACKGROUND OF THE INVENTION

Several approaches have been developed for detection of analytes in a biological sample for routine diagnostics in diagnostic laboratories via for instance immunoassays.

In essence, immunoassays can be performed in a liquid-based assay using e.g. a plastic well (e.g. in a 96 well plate) with certain immunoreagents. Alternatively, the immunoassay detection can be done on membrane based system (e.g. test strips). The plastic well-based system generally is limited to the detection of a single analyte while recent membrane-based assays have been developed to enable the simultaneous detection and/or quantitation of different analytes per assay.

Commercially available test strips are generally coated with a series of different ligands, each of which being able to independently bind a respective specific analyte in a biological sample. However, the combination of ligands available on one particular multi-parametric test strip does not always match with the set of analytes to be tested for a patient. Therefore, different test strips need to be used in order to test all the required analytes for the patient. However, not all of the ligands available on the different test strips are useful for a certain patient and some of the ligands available on the different test strips are even redundant. Consequently, the use in routine laboratories of multi-parametric test strips most often lacks flexibility to match with the exact analysis required for each particular patient and remains generally severely limited for economical reasons.

In view thereof, there remains a need in the art for further and/or improved multi-parametric systems for the detection of analytes in a biological sample.

### SUMMARY OF THE INVENTION

The present inventors have now found a kit comprising a solid support unit strip and a holder, addressing one or more of the above-mentioned problems of the prior art.

Hence, a first aspect of the invention relates to a kit for preparing a custom-made test strip for detecting at least two analytes, the kit comprising at least one solid support unit (SSU) strip and at least one holder, wherein the SSU strip comprises multiple, tandemly arranged solid support units, wherein each solid support unit is identical within the SSU strip and each solid support unit is detachable from the SSU strip, and wherein the holder comprises a body having a generally longitudinal shape, wherein said body is provided with at least two receiving regions, wherein each receiving region is configured to receive a solid support unit.

The kit embodying the principles of the present invention advantageously allows the user or consumer such as a medical laboratory to make a test strip adapted to the patient, or in other words to tailor the test strip towards the required analytes, for instance as prescribed by the medical practitioner. Such a kit thus allows testing only the required analytes without having to test irrelevant and/or redundant analytes. Furthermore, the present kit advantageously reduces the number of test to be performed and the number of results to be analyzed, validated and interpreted, thereby increasing the speed of testing, reducing the amount of sample needed, lowering the impact on the environment by reducing the required reagents and consumables, and hence reducing the costs.

As mentioned above, the present kit allows the user or consumer such as a medical laboratory to test only those analytes that were prescribed by a medical practitioner which might also be important from a legal point of view. Indeed, the present kit allows testing and reporting of only those analytes which are prescribed by the medical practitioner and/or which are refunded to the patient.

From a manufacturing point of view, the present kits are also economically advantageous because they allow the manufacturer to screen the quality of each SSU strip separately. For example, the present kit allows the manufacturer to screen the quality of the coating of a ligand on the SSU strip separately from the quality of the coating of other ligands. On the contrary, in existing multi-parametric test strips, several ligands are coated on a single test strip and hence, when the coating of one of the ligands falls outside the required quality specifications range, the complete multi-parametric test strip (including the coated ligands) has to be discarded.

A second aspect relates to a solid support unit (SSU) strip for preparing a custom-made test strip for detecting at least two analytes, the SSU strip comprising multiple, tandemly arranged solid support units, wherein each solid support unit is identical within the SSU strip and each solid support unit is detachable from the SSU strip.

A further aspect of the invention relates to a holder for holding at least two solid support units, the holder comprising a body having a generally longitudinal shape, wherein said body is provided with at least two receiving regions, wherein each receiving region is configured to receive a solid support unit.

The SSU strip embodying the present invention and the holder embodying the present invention advantageously allow the creation of a custom-made multi-parametric test strip adapted to the needs of the user, for instance adapted to the patient. The user or consumer such as a medical laboratory may select two or more different SSU strips for a patient in order to create a patient-personalized test strip in complete accordance with the required analysis. The number of diagnostic parameters to be used in the test strip is thereby rationalized, since the detection of irrelevant and/or redundant analytes is avoided.

Hence, a further aspect of the invention relates to the use of at least two different SSU strips as defined herein and a holder as defined herein, for preparing a custom-made test strip for detecting at least two analytes.

A further aspect of the invention provides a method for preparing a custom-made test strip for detecting at least two analytes in a biological sample, comprising the steps of: (a) providing at least two different solid support units, (b) providing a holder as defined herein, and (c) loading at least two receiving regions of the holder each with a different solid support unit.

In certain embodiments of the kit as taught herein, the kit comprises at least two different SSU strips.

In certain embodiments of the kit or the strip, as taught herein, each solid support unit comprises an immunoassay. In certain embodiments of the kit or the strip, as taught herein, the SSU strip comprises at one end a coupling element for coupling to a dispenser configured to dispense one solid support unit at a time. In certain embodiments of the kit or the strip, as taught herein, the SSU strip is comprised in a dispenser configured to dispense one solid support unit at a time.

In certain embodiments of the kit or the holder, as taught herein, each receiving region comprises a compartment in the body of the holder having a receptive opening for insertably receiving the solid support unit, and wherein the compartment is configured to mechanically secure the solid support unit. In certain embodiments of the kit or the holder, as taught herein, the receptive opening is a slot in a longitudinal side wall of the body, which slot is configured for slidably receiving the solid support unit and connects the outside of the body to an interior void space of the compartment. In certain embodiments of the kit or the holder, as taught herein, each receiving region comprises an adhesive bonding surface on the body of the holder configured to bond adhesively with the solid support unit. In certain embodiments of the kit or the holder, as taught herein, at least two receiving regions are each provided with a solid support unit.

In certain embodiments of the method as taught herein, each solid support unit comprises an immunoassay. In certain embodiments of the method as taught herein, the method for detecting at least two analytes in a biological sample further comprises the steps of: (d) loading a biological sample in a sample chamber of said holder, (e) inserting the loaded holder of step (d) in a container comprising at least one fluid, and (f) detecting at least two analytes in the biological sample.

These and further aspects and embodiments of the invention are hereunder further explained in the following sections and in the claims, and illustrated by non-limiting figures. The reference numbers relate to the hereto-annexed figures.

### BRIEF DESCRIPTION OF THE DRAWINGS

**FIG. 1A** schematically represents a SSU strip according to an embodiment of the invention.
**FIG. 1B** schematically represents a side view of a SSU strip according to an embodiment of the invention.
**FIG. 1C** schematically represents an enlarged side view of an end part of a SSU strip according to an embodiment of the invention.
**FIG. 1D** schematically represents an enlarged side view of an end part of a SSU strip according to an embodiment of the invention.
**FIG. 2** schematically represents a holder according to an embodiment of the invention.
**FIG. 3** schematically represents a holder according to another embodiment of the invention.
**FIG. 4A** schematically represents a front view of an enlarged part of a holder according to an embodiment of the invention.
**FIG. 4B** schematically represents a rear view of an enlarged part of a holder according to an embodiment of the invention.
**FIG. 4C** schematically represents a side view of an enlarged part of a holder according to an embodiment of the invention.
**FIG. 4D** schematically represents a side view of an enlarged part of a holder according to an embodiment of the invention.
**FIG. 4E** schematically represents a side view of a cross-section along the longitudinal axis A-A' of an enlarged part of a holder according to an embodiment of the invention.
**FIG. 4F** schematically represents a side view of a cross-section along the longitudinal axis A-A' of an enlarged part of a holder according to an embodiment of the invention.
**FIG. 5** schematically represents a dispenser according to an embodiment of the invention.
**FIG. 6** is a plan view of a transmission element engaged within first and second longitudinal members.
**FIG. 7** is a schematic view of a detail of a recess of a first or second longitudinal member.
**FIG. 7A** indicates the angle α^{r} of the pushing surface (PS) of a recess of a first or second longitudinal member.
**FIG. 7B** indicates the angle β^{r} of the sliding surface (SS) of a recess of a first or second longitudinal member.
**FIG. 8** is a schematic view of a detail of a pawl of a transmission element.
**FIG. 8A** indicates the angle α^{l} of the pushing surface (PS) of a pawl of a transmission element.
**FIG. 8B** indicates the angle β^{l} of the sliding surface (SS) of a pawl of a transmission surface.
**FIG. 9 to 12** schematically represents a method according to an embodiment of the invention for dispensing a solid support unit from a dispenser according an embodiment of the invention.
**FIG. 13** schematically represents a dispenser according to an embodiment of the invention.
**FIG. 14A** schematically represents an arrangement comprising a holder according to an embodiment of the present invention, a dispenser according to an embodiment of the invention, and a loading device according to an embodiment of the present invention.
**FIG. 14B** schematically represents a view of the arrangement of **FIG. 14A** along the longitudinal axis of the dispenser.

### DETAILED DESCRIPTION OF THE INVENTION

Unless defined otherwise, all technical and scientific terms used herein have the same meaning as is commonly understood by one of skill in the art. All publications referenced herein are incorporated by reference thereto.

The articles 'a' and 'an' are used herein to refer to one or to more than one, i.e. to at least one of the grammatical object of the article.

Throughout this application, the term 'about' is used to indicate that a value includes the standard deviation of error for the device or method being employed to determine the value.

The recitation of numerical ranges by endpoints includes all integer numbers and, where appropriate, fractions subsumed within that range (*e.g.* 1 to 5 can include 1, 2, 3, 4 when referring to, for example, a number of elements). The recitation of end points also includes the end point values themselves (*e.g.* from 1.0 to 5.0 includes both 1.0 and 5.0).

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

In the following detailed description of the invention, reference is made to the accompanying drawings that form a part hereof, and in which are shown by way of illustration only of specific embodiments in which the invention may be practiced. It is to be understood that other embodiments may be utilized and structural or logical changes may be made without departing from the scope of the present invention.

The present inventors have realized that a kit comprising at least one solid support unit (SSU) strip and at least one holder allows for the creation of a custom-made test-strip by the user or consumer.

Accordingly, in a first aspect, the present invention provides a kit for preparing a test strip for detecting at least two (different) analytes, the kit comprising at least one solid support unit (SSU) strip and at least one holder, wherein the SSU strip comprises multiple, tandemly arranged solid support units, wherein each solid support unit is identical within the SSU strip and each solid support unit is detachable from the SSU strip, and wherein the holder comprises a body having a generally longitudinal shape, wherein said body is provided with at least two receiving regions, wherein each receiving region is configured to receive a solid support unit. The analytes are present in a sample.

In an embodiment, the kit as taught herein may comprise at least two different SSU strips as defined herein. For example, the kit as taught herein may comprise at least three, at least four, at least five, at least six, at least seven, or at least eight different SSU strips as defined herein.

Such a kit comprising at least two SSU strips advantageously allows for the creation of a custom-made multi-parametric test-strip by the user or consumer for the detection of at least two analytes. The analytes may be different.

In an embodiment, the kit as taught herein may comprise two or more SSU strips as defined herein, each SSU strip being configured for a different assay.

In an embodiment, the kit as taught herein may comprise one or more holders as defined herein, each holder being configured for holding at least two solid support units.

In an embodiment, the kit as taught herein may further comprise at least one dispenser configured to dispense one solid support unit at a time.

The term "kit" or "kit of parts", as used herein, refers to any combination of apparatus that can be used to prepare a test strip as defined herein. The kit of the invention can further include any additional devices, containers, reagents, buffers, and/or excipients, as required described herein or known in the art, to practice a method of the invention. Other kit elements can include casings for packaging, packaging materials, solutions for use in the assay, and the like.

A set of instructions for directing a user in the use of the SSU strips, holders, kits, uses, or methods according to the invention will also be typically included.

In a second aspect, the present invention provides a solid support unit (SSU) strip for preparing a test strip for detecting at least two analytes, the SSU strip comprising multiple, tandemly arranged solid support units, wherein each solid support unit is identical within the SSU strip and each solid support unit is detachable from the SSU strip.

The SSU strip and hence a solid support unit has an upper side and an under side. The upper side and under side are essentially planar. The upper side and under side are essentially parallel. The upper side of the SSU provides a surface onto which a ligand can be immobilized. The upper side may be formed, for instance, from a solid support material or may be an assay membrane that is attached to a backing layer.

Each solid support unit may comprise an identification element. The identification element allows the user to visually inspect or verify which type of solid support unit(s) has (have) been inserted into the holder. Each solid support unit may comprise an orientation or positioning element. The orientation or positioning element allows an automatic reading or interpretation system to identify a reference position from which the different positions of the discrete test areas (such as ligands, controls, standards) to be measured on the solid support unit can be extrapolated.

In an embodiment, the under side of the solid support unit may be provided with an identifier. The identifier is preferably optically readable. The identifier may be a visual marking such as a bar code or quick response (QR) code.

In certain embodiments, the SSU strip may have pushability.

The term "pushability", as used herein, refers to the characteristic of the SSU strip which allows slidable insertion of a solid support unit into the holder without buckling.

Advantageously, the SSU does not need to be transparent, hence ligands that would not normally bind to a convention transparent microplate well can be utilised.

In certain embodiments, the SSU strip and hence a solid support unit may be substantially made of a solid support material onto which a ligand can be immobilized. In certain embodiments, the solid support material may be any suitable rigid material onto which a ligand can be immobilized. In certain embodiments, the solid support material may be substantially made of a non-chemically-activated material onto which a ligand can be immobilized. In certain embodiments, the solid support material may be substantially made of a chemically-activated material onto which a ligand can be immobilized. It may contain no assay membrane.

In certain embodiments, the solid support material may a polymeric material or glass. In certain embodiments, the solid support material may be substantially plastic, such as for example polystyrene, polypropylene or polycarbonate. The solid support material can have a substantially uniform level of opacity and can be of any uniform color. It is preferable opaque. This is particularly advantageous when the under side of the solid support unit is provided with an identifier, such as a barcode or QR code, which can then be scanned while being held in the receiving regions of the holder.

In certain embodiments, the SSU strip and hence a solid support unit may comprise a membrane supported on a backing layer. In certain embodiments, the SSU strip and hence a solid support unit may comprise a backing layer and an assay membrane, wherein the assay membrane is provided on the backing layer. The backing layer is on the under side of the SSU strip (and hence the solid support unit), and the assay membrane is on the upper side of the SSU strip (and hence the solid support unit).

The terms "membrane" and "assay membrane" may be used interchangeably herein. The ligand can be immobilized on the "membrane" or "assay membrane".

The terms "backing layer" or "solid backing" may be used interchangeably herein.

In certain embodiments, the backing layer may be substantially made of a polymeric material or glass. In certain embodiments, the backing layer may be substantially made of plastic, preferably polyester.

In certain embodiments, the assay membrane may be substantially made of cellulose, preferably nitrocellulose. In certain embodiments, the assay membrane may be substantially made of nylon or vinyl, preferably polyvinyl.

In certain embodiments, the solid support unit may comprise at least one ligand. In certain embodiments, the solid support unit may comprise at least one ligand which is specific to at least one analyte to be detected.

In certain embodiments, the solid support unit may comprise at least one ligand which is immobilized onto the solid support unit, for instance on the upper side of the solid support material. In certain embodiments, the SSU strip and hence the solid support unit may comprise at least one ligand which is immobilized onto the assay membrane.

In this context, the terms "fixed", "coated", "immobilized", or "bound" may be used interchangeably herein. When the solid support unit is described as comprising a ligand, positive control, or negative control, it is meant that the ligand, positive control, or negative control is "fixed", "coated", "immobilized", or "bound" to a surface of the solid support unit, preferably to the upper side.

In certain embodiments, the at least one ligand may be a protein, a modified protein, a peptide, a nucleic acid (such as a deoxyribonucleic acid or a ribonucleic acid), a hapten, an antigen, an antibody, or a metabolite of any of these substances, as well as any other compound (either natural or synthetic) which may be of diagnostic interest and which has a specific ligand-binding partner (i.e., the receptor moiety of the ligand-receptor assay). For example, the ligand may be selected from the group consisting of an antibody, an antigen, a protein, a hapten, and a nucleic acid.

In certain embodiments, the ligand may be selected from:
- an antibody comprising a monoclonal antibody raised against one or more antigens of the sample such as animal or human proteins, including antibodies, or a hapten or other organic or inorganic molecules;
- an antigen capable of specifically interacting with one or more antibodies of the sample;
- a protein, such as protein A, protein G, or protein L able to bind the Fc fragment of certain immunoglobulins;
- a natural or synthetic amino acid sequence able to make specific interactions with certain organic molecules, for example a synthetic peptide, a hapten or other organic or inorganic molecules;
- a natural or synthetic nucleotide sequence, such as for example single stranded or double stranded DNA or RNA, able to make specific interactions with a complementary nucleotide sequence, or with certain organic molecules, for example a synthetic peptide, a hapten or other organic or inorganic molecules; or,
- a combination of any two, any three, any four, or any five of the aforementioned ligands.

In certain embodiments, the ligand may be selected from the group consisting of an antigen, an antibody, a protein, a hapten, and a nucleic acid. In certain embodiments, the ligand may be an antigen. In certain preferred embodiments, the ligand may be an auto-antigen.

As used herein, the term "ligand" refers to an agent capable of detecting an analyte, for instance in a sample (such as a biological sample).

As used herein, the term "analyte" refers to an agent capable of binding a ligand, for instance fixed on a solid support unit (such as on an assay membrane).

The terms "analyte" and "ligand", as used herein, refer to members of any specific binding pair of which the ligand is immobilized on a solid support unit (such as an assay membrane), and the analyte is present in a sample (such as a biological sample) to be contacted with the solid support unit (such as the assay membrane). The ligand therefore refers to any capturing or catching agent immobilized on the solid support unit (surface) and the analyte refers to any specific binding partner thereto. The ligand and the analyte can be the same type of molecule depending on the concept of the assay.

The immobilization may occur through an interaction selected from different types of interactions such as ionic binding, covalent binding or hydrophobic interactions. The number of ligands immobilized on a solid support unit (such as on an assay membrane of the solid support unit) depends on the test. The number of ligands immobilized on a solid support unit (such as on an assay membrane of the solid support unit) may range from 1 to hundreds depending on the dimension of the solid support unit and the surface occupied by each ligand.

In certain embodiments, the solid support unit may comprise at least one ligand. For example, the solid support unit may comprise one or more ligands, such as at least two, at least three, at least four, at least five, at least six, at least seven, or at least eight ligands.

The solid support unit, in particular the upper side, can comprise at least one test area or assay area. Preferably, said solid support unit, in particular the upper side, comprises at least 3 assay areas, more preferably at least 6 assay areas, more preferably at least 9 assay areas. Each assay area can be designed to specifically detect one analyte in the sample.

The terms "test area" or "assay area", as used herein, refers to a discrete area on the solid support unit. A test area is preferably disposed with ligand, *i.e.* ligand is immobilized on the solid support unit in an area limited to the test area. It is preferably coated with ligand. The test area may be disposed with one ligand or with 2 or more different ligands. The test area may be a spot or line, or may have any form which allows detection such as visual detection.

In certain embodiments, the analyte may be any molecule for which the detection and/or measurement of the concentration are possible and for which a specific complementary molecule, a ligand, exists. The ligand and the analyte may for example be stereo-complementary which allows the fixation of the analyte on the solid support unit through the binding with a ligand which is easy accessible at the surface of the solid support unit. Without limitation, analytes that may be tested for and/or detected include drugs of abuse or their metabolites, analytes indicating the presence of an infectious agent or product of an infectious agent, allergen, pollutant, toxin, contaminant, analyte with diagnostic or medical value, antibody against any of the foregoing, and any combination thereof.

The terms "detecting" or "detection" generally refer to determining the presence or absence of an analyte in a sample, such as a biological sample. The recitation "detecting an analyte" may encompass measuring an analyte.

The terms "measuring" or "measurement" generally refer to determining the amount, quantity, or concentration of an analyte in a sample, such as a biological sample. The term "semi-quantitative measurement" generally refers to determining an approximation of the amount, quantity, or concentration of an analyte in a sample, such as a biological sample.

In certain embodiments, the analyte may be selected from the group consisting of an antibody, an antigen, a protein, a hapten, and a nucleic acid. In certain embodiments, the analyte may be an antibody. In certain preferred embodiments, the analyte may be an auto-antibody.

In certain embodiments, the solid support unit may be used to detect an analyte in a biological sample. The biological sample may be a body fluid such as serum, plasma, blood, cerebrospinal fluid, nasopharyngeal secretions, urine, semen, or saliva; food; water; culture supernatants or media; or excrement. In certain preferred embodiments, the biological sample may be selected from the group consisting of serum, plasma, and blood.

In certain embodiments, the solid support unit may comprise at least one ligand, wherein the ligand may be present in two or more different test areas at the same concentration on the solid support unit. For example, the same ligand may be present in duplicate (*i.e.,* two times), in triplicate (*i.e.,* three times), four times, five times, or six times, at the same concentration on the solid support unit. This advantageously allows calculating a mean value and/or standard deviation of the values which is recommended or even required for performing certain assays such as diagnostic assays.

In certain embodiments, the solid support unit may comprise at least one positive control. For example, the solid support unit may comprise one or more positive controls, such as at least two, at least three, at least four, at least five, at least six, at least seven, or at least eight positive controls present in different test areas. The positive controls may be of the same composition at the same concentration, of the same composition at different concentrations, of different composition at the same concentration, or of different composition at different concentrations. In certain embodiments, the solid support unit may comprise at least one positive control, wherein the positive control may be present in two or more different test areas at the same concentration on the solid support unit. For example, the positive control may be present in duplicate (*i.e.,* two times), in triplicate (*i.e.,* three times), four times, five times, or six times at the same concentration on the solid support unit. This advantageously allows calculating a mean value and/or standard deviation of the values which is recommended or even required for performing certain assays such as diagnostic assays.

The term "positive control", as used herein, refers to a test area where a phenomenon (or 100% effect or 100 units) is expected. That is, a positive control ensures that there is an effect when there should be an effect, for instance by using a ligand that is already known to produce that effect with the sample under test. A positive control may be used to assess test validity, such as to assess the presence of the sample.

The terms "positive control" or "sample control" may be used interchangeably.

In certain embodiments, the solid support unit may comprise at least one negative control. For example, the solid support unit may comprise one or more negative controls, such as at least two, at least three, at least four, at least five, at least six, at least seven, or at least eight negative controls present in different test areas. The negative controls may be of the same composition at the same concentration, of the same composition at different concentrations, of different composition at the same concentration, or of different composition at different concentrations. In certain embodiments, the solid support unit may comprise at least one negative control, wherein the negative control may be present in two or more different test areas at the same concentration on the solid support unit. For example, the negative control may be present in duplicate (*i.e.,* two times), in triplicate (*i.e.,* three times), four times, five times, or six times at the same concentration on the solid support unit. This advantageously allows calculating a mean value and/or standard deviation of the values which is recommended or even required for performing certain assays such as diagnostic assays.

The term "negative control", as used herein, refers to a test area where no phenomenon (zero effect or 0% effect) is expected. That is, a negative control ensures that there is no effect when there should be no effect, for instance by using a ligand that is already known to produce no effect. A negative control may be used to assess the cut-off or normalisation value.

The terms "negative control" or "cut-off control" may be used interchangeably.

In certain embodiments, the solid support unit may comprise at least one ligand, at least one positive control, and at least one negative control. Such a solid support unit advantageously allows semi-quantitative measurement of an analyte binding to the at least one ligand. The at least one positive control and the at least one negative control allow the semi-quantitative measurement of an analyte binding to the at least one ligand. It is thus understood that the at least one positive control and the at least one negative control may be standards. The negative control may be a zero unit standard. The positive control may be a 100 units standard.

In certain embodiments, the solid support unit comprises at least one ligand, at least one positive control, and at least one negative control, wherein the at least one ligand, the at least one positive control, and the at least one negative control are each present in two or more different test areas at the same concentration on the solid support unit. For example, the at least one ligand, the at least one positive control, and the at least one negative control may be present in duplicate (*i.e.,* two times), in triplicate (*i.e.,* three times), four times, five times, or six times at the same concentration on the solid support unit. This advantageously allows calculating a mean value and/or standard deviation of the values which is recommended or even required for performing certain assays such as diagnostic assays.

In certain preferred embodiments, the solid support unit comprises one ligand, one positive control, and one negative control. In certain preferred embodiments, the solid support unit comprises one ligand, one positive control, and one negative control, wherein the ligand, the positive control, and the negative control are present in two or more different test areas at the same concentration on the solid support unit. For example, the ligand, the positive control, and the negative control are present in duplicate (*i.e.,* two times), in triplicate (*i.e.,* three times), four times, five times, or six times at the same concentration on the solid support unit. In certain preferred embodiments, the solid support unit comprises one ligand, one positive control, and one negative control, wherein the ligand, the positive control, and the negative control are present in triplicate at the same concentration on the solid support unit (such as on the assay membrane of a solid support unit). This advantageously allows calculating a mean value and/or standard deviation of the values which is recommended or even required for performing certain assays such as diagnostic assays.

In certain embodiments, the solid support unit may comprise at least two standards disposed in different test areas. Such a solid support unit advantageously allows a semi-quantitative measurement of the ligand. In certain embodiments, the solid support unit may comprise at least three standards disposed in different test areas. Such as solid support unit advantageously allows a quantitative measurement of the ligand. In certain embodiments, the solid support unit may comprise at least three standards for a calibration curve, wherein the read-out (e.g. colour) of the standards increases with increasing concentration. For example, the solid support unit as defined herein may comprise three of more standards disposed in different test areas. For example, the solid support unit as defined herein may comprise at least four, at least five, at least six, at least seven, at least eight, at least nine, at least ten, at least eleven, or at least twelve standards disposed in different test areas for a calibration curve, wherein the read-out (e.g. colour) of the standards increases with increasing concentration.

In certain embodiments, the solid support unit may comprise a control for validation of correct handling. In certain embodiments, the solid support unit may comprise at least three controls disposed in different test areas such as at least one control for validation of correct handling, at least one control for validation of the performance and/or type of tracer used, and at least one control for validation of the signal development system.

The ligands and/or standards and/or controls may be applied to different test areas (such as spots) to the solid support unit. In certain embodiments, the solid support unit may comprise test areas in a matrix array. In certain embodiments, the solid support unit may comprise two times two (*i.e.,* 2 x 2), three times three (*i.e.,* 3 x 3), four times four (*i.e.,* 4 x 4), or five times five (*i.e.,* 5 x 5) test areas in a matrix array. For example, the solid support unit may comprise two times two (*i.e.,* 2 x 2) spots, three times three (*i.e.,* 3 x 3) spots, four times four (*i.e.,* 4 x 4) spots, or five times five (*i.e.,* 5 x 5) spots. For example, the solid support unit as defined herein may comprise one ligand, one positive control, and one negative control, wherein the ligand, the positive control, and the negative control are present in triplicate at the same concentration on the solid support unit, and wherein the solid support unit may comprise three times three (*i.e.,* 3 x 3) test areas in a matrix array. The test areas (such as spots) should be machine readable, preferably, optically readable.

The standards, the positive control, and the negative control may be selected from:
- an antibody, comprising a monoclonal antibody raised against one or more antigens present in the sample or in any reagent required to process the assay, for example animal or human proteins, including antibodies, or a hapten or other organic or inorganic molecules;
- an antigen, specific for one or more antibodies present in the sample or in any reagent required to process the assay;
- a protein, such as protein A, protein G, or protein L able to bind the Fc fragment of certain immunoglobulins;
- a natural or synthetic amino acid sequence able to make specific interactions with certain organic molecules, for example a synthetic peptide, a hapten or other organic or inorganic molecules;
- a natural or synthetic nucleotide sequence, such as for example single stranded or double stranded DNA or RNA, able to make specific interactions with a complementary nucleotide sequence;
- an enzyme or any organic or inorganic molecule, linked or not to another organic or inorganic molecule, able to generate a detectable signal, either in the presence of a signal-generating source or naturally by its nature, for example alkaline phosphatase or peroxidase, fluorescent dyes, colloidal metal particles or light-emitting molecules; or
- a combination of any two, any three, any four, any five, or any six thereof.

In certain embodiments, the assay may be an immunoassay. In certain embodiments of the kits, SSU strips, uses, or methods, as taught herein, each solid support unit may comprise an immunoassay.

The term "immunoassay" as used herein refers to an assay comprising at least one antigen specific to at least one antibody to be detected, or comprising at least one antibody specific to at least one antigen to be detected.

In certain embodiments, the immunoassay may comprise at least one antigen specific to at least one antibody to be detected. In certain preferred embodiments, the immunoassay may comprise at least one auto-antigen specific to at least one auto-antibody to be detected.

As mentioned above, each solid support unit may comprise a backing layer and an assay membrane provided on the backing layer. In certain embodiments, the assay membrane may be an immunoassay membrane. Hence, in certain embodiments, each solid support unit may comprise an immunoassay membrane.

The term "immunoassay membrane" as used herein refers to an assay membrane comprising at least one antigen specific to at least one antibody to be detected, or comprising at least one antibody specific to at least one antigen to be detected.

In certain embodiments, the immunoassay membrane may comprise at least one antigen specific to at least one antibody to be detected. In certain preferred embodiments, the immunoassay membrane may comprise at least one auto-antigen specific to at least one auto-antibody to be detected.

The term "auto-antigen" as used herein refers to an antigen (such as a protein or complex of proteins or DNA or RNA) that is recognized by the immune system of patients having an autoimmune disease, where the antigen would, under healthy conditions, not be the target of the immune system. Due to for instance genetic and/or environmental factors, the normal immunological tolerance an auto-antigen has been lost in patients having an autoimmune disease.

In certain embodiments the antigen may be selected from the group comprising or consisting of double stranded DNA (dsDNA), Sm (D and/or BB' proteins from U1-U6 snRNP complexes), RNP (68kD and/or A and/or C proteins from U1-snRNP complex), SSA/Ro (60kD Sjogren Syndrome antigen A), SSB/La (50 kD Sjogren antigen B), JO-1 (Histidyl-tRNA-synthetase), and Scl-70 (DNA Topoisomerase I).

As mentioned before, the present invention provides a SSU strip comprising multiple, tandemly arranged solid support units, wherein each solid support unit is identical within the SSU strip and each solid support unit is detachable from the SSU strip.

A solid support unit may be readily detached from the SSU strip, i.e. a solid support unit may be detached from the SSU strip (*e.g.* by the user or consumer) manually without the need for tools, or with relatively simple tools, such as a utility knife. The terms "detachable", "non-permanent", or "removable" can be used interchangeably herein. For instance, a solid support unit may be detached from the SSU strip (*e.g.* by the user or consumer) by tearing one solid support unit of the SSU strip or by breaking one solid support unit of the SSU strip, or a solid support unit may be detached from the SSU strip (*e.g.* by the user or consumer) by cutting one solid support unit of the SSU strip for instance with a utility knife.

In certain embodiments, the solid support unit may be tearable or breakable from the SSU strip.

In certain embodiments, the solid support units (i.e. two neighbouring solid support units) may be at least partly associated or connected. For example, the solid support units as defined herein (i.e. two neighbouring solid support units) may be associated or connected by at least part of the backing layer.

In certain embodiments, the assays of the solid support units may be completely separated for instance by means of a pre-scoring or groove between the solid support units.

In certain embodiments, the membranes of the solid support units (i.e. two neighbouring solid support units) may be completely separated for instance by means of a pre-scoring or groove between the solid support units. In certain embodiments, the backing layer of the solid support units (i.e. two neighbouring solid support units) may be partially separated for instance by means of a groove between the solid support units.

The association between adjacent solid support units is meant to be detachable, i.e., the association can be easily removed or disrupted by a user or consumer. Typically, the association can be removed or disrupted manually by the user without the need for tools, or with relatively simple tools, such as a utility knife. For instance, the association between the solid support units can be easily removed or disrupted by the user by tearing or breaking one solid support unit of the SSU strip, or the association between the solid support units can be easily removed or disrupted by the user by cutting one solid support unit of the SSU strip for instance with a utility knife.

Alternatively, the solid support unit may be detached from the SSU strip (*e.g.* by a user or consumer) by means of a loading device as described herein.

The SSU strip may comprise or contain two or more solid support units, for instance the SSU strip may comprise or contain 2, 3, 4, 5, 6, 7, 8, 9, 10, 11, 12, 13, 14, 15, 16, 17, 18, 19, 20, 21, 22, 23, 24, 30, 40, or 50 solid support units. Preferably, the SSU strip comprises or contains 24 solid support units. The SSU strip may have a longitudinal body. The SSU strip may be provided as a linear strip; this configuration may be suited for manual dispensing using a pen-like dispenser. The SSU strip may be provided on a roll; this configuration may be suited for automated dispensing.

In certain embodiments of the kits, SSU strips, uses, or methods, as taught herein, the SSU strip may comprise at one end a coupling element for coupling to a dispenser configured to dispense one solid support unit at a time. The coupling element may be any element allowing coupling to a dispenser configured to dispense one solid support unit at a time.

In certain embodiments of the kits, SSU strips, uses, or methods, as taught herein, the SSU strip may be comprised in a dispenser configured to dispense one solid support unit at a time.

A further interrelated aspect of the invention provides a holder for holding at least two solid support units, the holder comprising a body having a generally longitudinal shape, wherein said body is provided with at least two receiving regions, wherein each receiving region is configured to receive (and hold) a solid support unit.

The body may define a longitudinal (A-A') axis. The body of the holder has a front side from which the solid support units held in the receiving regions can be viewed or read, a rear side opposite to the front side, a top end at one longitudinal end, and a base end at the other longitudinal end. Connecting the front side to the rear side are a pair of longitudinal side edges, and a pair of end side edges. The holder preferably is adapted for robotic manipulation, for instance, for automated sample processing.

The receiving regions are arranged in a longitudinal direction along the body. Each receiving region is configured to receive a solid support unit. Each receiving region may be configured to retain a solid support unit. Each receiving region is spatially separated from any neighbouring or adjacent receiving region. Such a holder advantageously allows that each solid support unit (held in a receiving region) is separated from each adjacent solid support unit (held in an adjacent receiving region). Specifically, a receiving region is spatially separated from the next receiving region. Each receiving region may be a delimited area arranged along the longitudinal axis of the body. Each delimited area may be spatially separated from any neighbouring receiving region by a portion of the body. The delimitation may provide an indication to the user about the position of each solid support unit. The delimitation may be substantially complementary in size and shape with a solid support unit.

There may be two or more receiving regions, such as two, three, four, five, six, seven, or eight receiving regions, in a holder. Preferably, there may be six receiving regions in a holder. The receiving regions may be arranged along the front side of the body. The receiving regions may be arranged within the body.

In certain embodiments, the holder is provided with at least one empty receiving region. Preferably, the holder is provided with all receiving regions empty, *i.e.* devoid of solid support units. In certain embodiments, the holder is provided with empty receiving regions.

In certain embodiments of the kits, holders, uses, or methods, as taught herein, at least two receiving regions (of the at least two receiving regions) each hold a solid support unit. In certain embodiments, two or more receiving regions (of the at least two receiving regions) may each hold a solid support unit.

The holder may further be provided with a sample chamber. The sample chamber may be provided anywhere in the body, but is preferably located at the base end of the body. The sample chamber comprises a principal sample chamber opening that connects the outside of the body to the chamber void. Preferably, the principal sample chamber opening is on the front side of the body.

The sample chamber may comprise a secondary opening that connects the chamber void space to a side edge of the body, preferably to the side edge at the base end. The secondary opening may have a central axis (running perpendicular to the surface of the opening), such that the axes of said two openings are not co-axial.

The sample chamber is spatially separated from any receiving regions. Preferably the sample chamber has one neighbouring receiving region. The sample chamber is preferably separated from any neighbouring receiving regions by a non-detachable barrier. The barrier is preferably fluid impermeable.

The terms "non-detachable barrier", "permanent barrier" or "non-removable barrier" can be used interchangeably herein and generally refer to a barrier which can not be separated or disconnected from its association with another means such as for instance with the body of holder.

The terms "fluid-impermeable barrier" or "impermeable barrier" generally refer to a barrier not permitting passage of a fluid such as for instance of a fluidic biological sample.

The non-detachable barrier separating the sample chamber from the neighbouring receiving region may be a wall of the sample chamber or a part of said wall. Such barrier advantageously allows retention of a sample such as a biological sample in fluid isolation from the adjacent receiving region (and any other receiving region) prior to insertion of the solid support into a container comprising a fluid.

In an embodiment, the sample chamber is dimensioned such that the sample is held within the sample chamber by means of capillarity and/or by means of the surface tension of the sample. In this regard, the sample chamber can have any suitable geometric shape, including, for example, cubic, cuboids, triangular prism, cone, square-based pyramid, triangular based pyramid, hemispherical, or cylinderical. The chamber can define a void between 1 mm³ and 50 mm³, preferably between 1 mm³ and 40 mm³, preferably between 1 mm³ and 30 mm³, and more preferably between 1 and 25 mm³ in volume. For example, the chamber cavity may be sized such as to be able to hold about 20µl of liquid sample.

Preferably, the sample chamber has the shape of a trapezoidal prism defining six surfaces of which four surfaces are of essentially rectangular shape and two surfaces are of essentially trapezoidal shape.

The openings of the sample chamber may have any shape to allow introduction and/or release of said sample. Preferably, said opening is a round opening, a square opening or a rectangular opening. Preferably, when the chamber comprises two openings one on the front face and one on the bottom face of the support, the front face opening is round, and the bottom face opening is rectangular. Preferably the wall surrounding the round opening is slanted.

Neighbouring receiving regions are spatially separated along the body of the holder. In certain embodiments, spatial separation may be assisted by one or more spacing elements provided on the holder. A spacing element may be a passage that passes through the body, *i.e.* it connects the front side of the holder to the rear side of the holder. It preferably has a cuboid shape. The passage reduces the size and weight of the body. It provides a plurality of floodable zones, which assist in the immersion of the holder into a liquid.

Typically a spacing element is provided between a pair of neighbouring receiving regions, or between a receiving region and a sample chamber. Typically, one spacing element is provided between each adjacent pair of receiving regions, and between the base-end receiving region and the sample chamber. Where the side wall of a receiving region has an aperture that joins opposite sides of said side wall together, the aperture also joins the interior space of the receiving region with the cavity of the spacing element. This assists with an even exposure of the solid support unit to liquid during immersion and also with drainage after the holder has been withdrawn from the liquid.

The sample chamber is preferably spatially separated from any neighbouring receiving region(s) by a spacing element. Preferably the sample chamber has one adjacent spacing element. The sample chamber is separated from any adjacent spacing element. The sample chamber is preferably separated from any adjacent spacing element by the non-detachable barrier described above. The barrier is preferably fluid impermeable.

The body of the holder may be made from any suitable material having requisite properties of stability in analytical fluids, mechanical strength, and stiffness. Preferably the body is made of a polymeric material, such as, for example polystyrene, polypropylene or polycarbonate plastic. The body may have a substantially uniform level of opacity. It may be of any uniform colour or preferably can be see-through or transparent. This is particularly advantageous when the rear of a solid support unit is provided with camera-readable identification marking (*e.g.* a bar code or QR code), which can then be scanned while being held in the receiving region.

The body of the holder may further comprise a handle portion provided on the top end of the holder for holding, grasping, and/or moving the holder. Said upper or handle portion can be grasped manually or using an automated device.

According to one embodiment, the receiving region is an adhesive bonding surface on the front side of the body of the holder. The adhesive bonding surface is an area on the body that is configured to bond with a solid support unit using an adhesive bonding. The adhesive bonding surface may be suitable to receive and retain a solid support unit disposed with an adhesive backing. In such case, the adhesive bonding surface may or may not be disposed with adhesive. Where the adhesive bonding surface is not disposed with adhesive, it may be free from grease and/or have a gloss appearance which enhances adhesion. Alternatively, the adhesive bonding surface may be coated with an adhesive configured to bond with a solid support unit. In such case the solid support unit is provided without an adhesive backing. The adhesive backing where present may be protected with a peel-off backing sheet.

In a preferred embodiment, a receiving region may be a compartment within the body. A compartment may be disposed with a front opening that opens onto the front side of the body. The size and shape of the front opening is preferably configured to allow reading of the solid support unit (inserted in the holder), for instance, to observe any colour and/or intensity change. Preferably, the front opening has a rectangular shape of equal or unequal sides. It may have rounded corners. The size and shape of the front opening is configured to allow optical reading of the upper side of the solid support unit (such as the upper side of the assay membrane of the solid support unit), for instance, to measure the colour and/or intensity of the test areas. The compartment may be disposed with a rear wall, opposite to the front opening. The rear wall may contain a rear wall window that connects the compartment void space to the rear side of the body. The size and shape of the rear window is configured to allow optical reading of the underside of the solid support unit (inserted in the holder), for instance, to identify a printed identification code. The rear wall window may be an aperture, or may be a solid structure that is transparent. Where it is a solid structure, it may be made of the same material as the body of the holder. Preferably, the rear wall window has a rectangular shape of equal or unequal sides. It may have rounded corners.

Preferably, the solid support unit (inserted in the compartment of the holder) is supported by the rear wall.

The compartment may further comprise one or more side walls that connect the rear wall with the front opening. A side wall may contain an aperture that joins opposite sides of the wall together. The compartment has an interior void space.

The compartment is configured to receive an SSU. The compartment may be provided with a receptive opening configured to receive the SSU.

The receiving region may comprise a compartment in the body of the holder having a receptive opening configured for insertably receiving the solid support unit, and wherein the compartment is configured to mechanically secure the solid support unit. The receptive opening may be the aforementioned front opening. In such case, the solid support unit enters the compartment through the front opening. The solid support unit may be pressed into position, by applying force to the upper side of the SSU. It is preferably pressed against the rear wall of the compartment.

The receptive opening may be a slot in one longitudinal side edge of the holder that connects the outside of the body to the compartment interior space. The slot is preferably longitudinal. It is preferably longitudinal in the direction of the longitudinal length of the body. The size and shape of the slot is configured to slidably receive a solid support unit. The solid support unit may be slidably inserted into the compartment through the slot. The solid support unit may be slidably inserted into the compartment through the slot using a dispenser as described herein.

The compartment is configured to mechanically secure the solid support unit. The solid support unit may be held in place by friction provided by the interior walls of the compartment. The solid support unit may be held or secured in place by a frictional lock. Alternatively, or in addition, the solid support unit may be held in place by a solid support unit retaining element. The compartment may comprise a solid support unit retaining element configured to maintain a solid support unit after insertion in the compartment. The solid support unit retaining element may comprise one or more protrusions in the compartment side wall, under which the solid support unit slides during insertion. The protrusions prevent or reduce the solid support unit from lifting from the compartment rear wall. The protrusions may frictionally engage with the solid support unit to hold it in place.

The solid support unit retaining element may comprise one or more grooves in the compartment side wall, into which the solid support unit slides during insertion. The grooves also prevent or reduce the solid support unit from lifting from the compartment rear wall). The grooves may frictionally engage with the solid support unit to hold it in place.

The solid support unit may be held or secured in place by a mechanical lock or stop. The mechanical lock may be the solid support unit retaining element as defined herein. The mechanical stop may be a side wall of the holder. The mechanical lock may be an anchoring system such as a knot (engaging with a groove in the solid support unit), groove (engaging with a knot on the solid support unit), or a clamp. The solid support unit can be secured in place by the mechanical lock and/or mechanical stop.

As mentioned above, the present inventors have realized that a holder and an SSU strip can be combined to allow the user or consumer to prepare a custom-made test strip. Hence, a further aspect of the invention provides the use of at least two different SSU strips as defined herein and a holder as defined herein, for preparing a test strip for detecting at least two analytes. Preferably, the at least two different analytes are different.

Also provided herein is the use of a SSU strip as defined herein or a solid support unit as defined herein, for preparing a test strip for detecting at least two analytes.

Further provided herein is the use of a holder as defined herein, for preparing a test strip for detecting at least two analytes.

A further aspect relates to a custom-made test strip for detecting at least two analytes, the test strip comprising a holder as defined in herein and at least two solid support units as defined herein, wherein each of the at least two solid support units is held in a receiving region of the holder.

In other words, a further aspect of the invention provides a test strip for detecting at least two analytes, the test strip comprising a holder as defined herein and at least two solid support units as defined herein, wherein the at least two solid support units each occupy a receiving region of the holder.

The term "test strip", as used herein, refers to an assay strip prepared by the user or consumer, preferably a multi-parametric assay strip prepared by the user or consumer. Advantageously, the present test strip is custom-made, for example the present test strip may be adapted to the needs of the user of consumer, or the present test strip may be patient-personalized, e.g. adapted to the patient.

The terms "test strip", "assay strip", "test stick", or "assay stick" may be used interchangeably herein.

In certain embodiments, the test strip may comprise, consist essentially of, or consist of one holder as defined in herein and at least two solid support units as defined herein. For example, the test strip as taught herein may comprise, consist essentially of, or consist of one holder and two or more solid support units as defined herein, such as two, three, four, five, six, seven, or eight solid support units as defined herein, wherein each of the two or more solid support units is held in a receiving region of the holder. In a preferred embodiment, the test strip as taught herein may comprise, consist essentially of, or consist of one holder and six solid support units as defined herein, wherein each of the six solid support units is held in a receiving region of the holder.

A further aspect relates to a test strip obtainable by the methods as taught herein.

The present invention also provides a method for preparing a test strip, comprising the steps of: (a) providing at least two different SSU strips as defined herein, (b) providing at least one holder as defined herein, (c) depositing each SSU strip of the at least two different SSU strips in one receiving region of the at least one holder, and (d) detaching a solid support unit from each of the at least two different SSU strips, thereby loading at least two receiving regions of the at least one holder each with a different solid support unit.

In particular, the method for preparing a test strip may comprise the steps of: (a) providing at least two different SSU strips as defined herein, (b) providing at least one holder for holding at least two solid support units, the holder comprising a body having a generally longitudinal shape, wherein said body is provided with at least two receiving regions, wherein each receiving region is configured to receive a solid support unit, (c) inserting each SSU strip of the at least two different SSU strips in one receiving region of the at least one holder, and (d) detaching one solid support unit from each of the at least two different SSU strips, thereby loading at least two receiving regions of the at least one holder each with a different solid support unit.

The method may be performed manually or in an automated manner.

In a preferred embodiment, the step (c) may be performed by slidably inserting each SSU strip in a compartment of the holder, where the receiving region is a compartment as defined herein.

Step (d) may be performed by rotating each SSU strip relative to the holder. The SSU strip may be rotated by at least about 60° relative to the holder in order to break the solid support unit from the SSU strip. Preferably, the dispenser may be rotated by at least about 90°, at least about 135°, or at least about 180°, relative to the holder. The rotation of the SSU strip is around a longitudinal axis of the SSU strip.

Alternatively the method for method for preparing a test strip, may comprise the steps of: (a₁) providing at least two different solid support units as defined herein, (b₁) providing at least one holder as defined herein, and (c₁) loading at least two receiving regions of the at least one holder each with a different solid support unit. Steps (a₁), (b₁) and (c₁) replace steps (a), (b) and (c) above.

In particular, the method for preparing a test strip may comprise the steps of: (a₁) providing at least two different solid support units as defined herein, (b₁) providing at least one holder for holding at least two solid support units, the holder comprising a body having a generally longitudinal shape, wherein said body is provided with at least two receiving regions, wherein each receiving region is configured to receive a solid support unit, and (c₁) loading at least two receiving regions of the at least one holder each with a different solid support unit. In certain embodiments, the method may comprise the prior steps of: (a') providing at least two different SSU strips as defined herein, and (a") detaching one solid support unit from each of the at least two different SSU strips, thereby providing the at least two different solid support units.

The step (c₁) may be performed by manually loading each solid support unit in a receiving region of the holder. The step (c₁) may be performed by automated loading of each solid support unit in a receiving region of the holder.

In a preferred embodiment, the step (c₁) may be performed by (slidably) inserting each solid support unit in a receiving region (such as a compartment) of the holder.

In certain embodiments, the method may be for detecting at least two analytes in a sample, such as a biological sample.

In certain embodiments of the method for detecting at least two analytes in a biological sample, the method may further comprise the steps of: (d) loading a biological sample in a sample chamber of said holder, (e) inserting the loaded holder of step (d) in a container comprising at least one fluid, and (f) detecting at least two analytes in the biological sample.

In an embodiment, the method is an automated method.

In certain preferred embodiments of the method for detecting at least two analytes in a biological sample, the method comprises the steps of:
- providing at least two different solid support units as defined herein, wherein the solid support units each comprise at least one ligand which is specific to at least one analyte to be detected,
- providing a holder as defined herein,
- loading at least two receiving regions of the holder each with a different solid support unit,
- loading a biological sample in a sample chamber of said holder,
- inserting the loaded holder in at least one container comprising at least one fluid, thereby releasing said biological sample in said at least one fluid,
- contacting the biological sample in the fluid with said ligands, thereby binding at least one analyte in said biological sample with each ligand,
- labeling each analyte bound to each ligand, by means of a marker, and
- detecting said marker, thereby determining a signal intensity of said marker.

The method of the present invention can be used to detect specific analytes in biological samples, whereby the user or consumer can determine the combination of analytes to be detected. For instance, the method allows the user or consumer to detect a combination of analytes customized or personalized to a certain patient.

Preferably, immunological assays are envisaged. These immunological assays can be used for semi-quantitative or quantitative measurements of analytes, with or without the use of a machine for the automated detection. In an embodiment, the signal intensity of the marker is directly in proportion to the concentration of the analyte to be detected in the biological sample.

Preferably, the method according to the invention comprises immunodot assay. Suitable non-limiting examples of immunodot technology include kits allowing the detection of auto-antibodies in serum of patients, for instance the BlueDot technology (D-Tek, Mons, Belgium).

In an embodiment, the test strip as taught herein can be used in a method for detecting auto-antibodies in a biological sample (for example serum) of a patient, said method comprising the steps of:
- providing at least two different solid support units as defined herein, wherein the solid support units each comprise at least one auto-antigen which is specific to at least one auto-antibody to be detected,
- providing a holder as defined herein,
- loading at least two receiving regions of the holder each with a different solid support unit, thereby obtaining a test strip,
- loading the biological sample in a sample chamber of said holder,
- inserting said test strip in at least one container comprising at least one fluid, thereby releasing said biological sample in said at least one fluid,
- specifically binding of the auto-antibodies present in the sample of the patient with auto-antigens bound to each solid support unit of the test strip, thereby binding the auto-antibodies to each solid support unit,
- washing the solid support units and auto-antibodies bound thereto to remove the sample,
- addition of a conjugate, comprising a secondary antibody directed against the auto-antibodies and wherein said secondary antibody is linked with an enzyme, thereby obtaining auto-antibody complexes,
- washing the solid support units and auto-antibody complexes bound thereto to remove unbound secondary antibody,
- incubation of the solid support units and auto-antibody complexes bound thereto with a colorimetric substrate,
- binding of the colorimetric substrate with the enzyme bound to the secondary antibodies of the auto-antibody complexes, thereby developing a specific color.

The appearance of a specific color on the solid supports indicates the presence of auto-antibodies in the serum of the patient and therefore, the presence of an autoimmune disease.

The test strips can be interpreted, for example by eye, by comparing the obtained intensity for the analyte (for example auto-antibodies) with the intensity of a negative and/or positive control. If the intensity is higher than the negative control, the signal is considered as positive. If the intensity is equal or lower than the negative control, the signal is considered negative. The present invention allows qualitative detection, but furthermore provides semi-quantitative measurement of the concentration of the analytes present in the sample. Semi-quantitative measurements are possible because each solid support can contain a positive control. Quantitative measurements are possible because each solid support can contain standards.

The present invention also provides a dispenser configured for dispensing a solid support unit from a SSU strip as defined herein.

In particular, the present invention provides a dispenser configured for dispensing a solid support unit from a SSU strip as defined herein, said dispenser having a distal dispensing end and a proximal end opposite the distal end, wherein the dispenser comprises:
- a first longitudinal member provided with an edge at least partially serrated by a plurality of tandemly-arranged recesses,
- a fixed second longitudinal member, provided with an edge at least partially serrated by a plurality of tandemly-arranged recesses, positioned parallel to the first longitudinal member, whereby the first longitudinal member is in slidable relation with the second longitudinal member, and
- a transmission element provided with a coupling for attachment to the SSU strip, a pair of pawls configured for engaging with the serrated edges, said transmission element configured to transmit bidirectional slidable movement of the first longitudinal member to a unidirectional movement of the coupling.

Advantageously, the present dispenser is configured to advance one solid support unit at a time, which solid support unit can be inserted into the holder described herein. The dispenser has, at one longitudinal end, a distal (dispensing) end, through which the solid support unit is advanced for dispensing, and at the other end, a proximal end. The elements that make up the dispenser may similarly have a distal or proximal end that corresponds to their orientation with respect to the dispenser distal or proximal end.

In certain embodiments, the dispenser comprises a SSU strip as defined herein.

In certain embodiments, the transmission element, the first longitudinal member, and second longitudinal member are configured such that:
- the transmission element slips against the serrated edge of a proximally-sliding first longitudinal member,
- the transmission element engages the serrated edge of a distally-sliding first longitudinal member,
- the serrated edge of the second longitudinal member slips against a distally-sliding transmission element,
- the serrated edge of the second longitudinal member engages the transmission element forced in a proximal direction.

The dispenser comprises a first longitudinal member, a second longitudinal member, a transmission element. In fixed attachment to the first longitudinal member may be an actuating element such as a thumb pad, for controlling a sliding movement of the first longitudinal member. The dispenser may further comprise a longitudinal casing for housing one or more of the elements. The casing is typically disposed with a slot at the distal end through which the solid support unit is dispensed. The casing may have any suitable shape. For instance, the casing may be cylindrical.

The first longitudinal member (FLM) of the holder is provided with an edge that is at least partly serrated. By serrated, it is meant that it is disposed with a multitude of discrete recesses, tandemly arranged. The first longitudinal member is slidably mounted with respect to the second longitudinal member. The first longitudinal member is preferably slidably mounted with respect to the casing. The first longitudinal member is slidably movable between two stop positions, *i.e.* a forward position and a backward position. The forward position is closer to the distal end, while the backward position is closer to the proximal end. Moving the first longitudinal member from the backward position to the forward position advances the SSU strip by one solid support unit in the direction of the distal end.

The second longitudinal member (SLM) of the holder is also provided with an edge that is at least partly serrated. The serrated edge preferably contains recesses of the same size, shape and spacing as the first longitudinal member. The second longitudinal member is fixedly mounted with respect to the casing (*i.e.* is in fixed relation with the casing). The first and second longitudinal members are arranged parallel to each other, preferably in the casing. The serrated edges are preferably mutually adjacent.

The transmission element comprises a coupling for dismountable or non-dismountable attachment to a SSU strip as described herein. The coupling maintains the SSU strip in fixed relation to the transmission element. The transmission element is configured to transmit the forward movement by the first longitudinal member, but not the backward movement by the first longitudinal member, to the SSU strip. Specifically, the transmission element engages with the first longitudinal member and moves forward together with a forward moving first longitudinal member, but slips against first longitudinal member and remains essentially static with respect to a backwardly moving first longitudinal member. Said transmission element is hence configured to transmit a bidirectional slidable movement of the first longitudinal member to a unidirectional movement of the coupling. At least part of the transmission element bridges the gap between the mutually parallel-arranged first and second longitudinal members.

The transmission element preferably comprises a longitudinal body comprising a first and second pawl. A "pawl" is a "tooth" or a projection from the transmission element dimensioned to fit into and engage with a recess. The pawls are preferably arranged so that they are symmetrical with respect to a central longitudinal axis of the transmission element. The pawls preferably project outwards. The pawls are preferably sprung with respect to each other. Each pawl engages with the serrated edge of the first and second longitudinal members respectively. More specifically, a pawlengages with a recess of a serrated edge. The transmission element is preferably configured to apply a pushing force between the first and second longitudinal members, for instance, by means of a spring or compliant member. The pushing force maintains the transmission means in a fixed position between opposing recesses.

The serrated edge of the first and second longitudinal members is disposed with a plurality of recesses, tandemly arranged. This is akin to a rack of a rack-and-pinion gear, except the teeth of this rack, or more properly the recesses, are shaped to transmit forces to or from a transmission element in one direction of movement but not in the other direction. Each recess provides an index for positioning the transmission element. Hence, when the transmission element is advanced by one recess, it is effectively advanced by one index that concomitantly advances the SSU strip by one solid support unit. As the second longitudinal member is fixed with respect to the casing, the index can be read off recesses on the second longitudinal member.

The recess of a longitudinal member comprises, at the proximal end of the recess, a recess pushing surface (recess PS), and at the distal end, a recess sliding surface (recess SS). A recess has a generally asymmetrical V-shaped profile.

The pawl of a transmission element comprises, at the proximal end of the pawl, a pawlpushing surface (pawIPS), and at the distal end, a recess sliding surface (recess SS). The tip of a pawlhas a generally asymmetrical V-shaped profile.

The recess PS is adapted to engage with the pawlPS, such that force in a distal direction applied by the first longitudinal member is transmitted to the transmission element *via* recess PS - pawlPS interaction. Similarly, the recess PS of the second longitudinal member is adapted to engage with the pawlPS, such that force in a proximal direction applied by transmission element is transmitted to the second longitudinal member via the recess PS - pawlPS interaction.

The recess PS is configured to engage with the pawlPS; this engagement allows for the transfer of forces between the pawl and the recess.

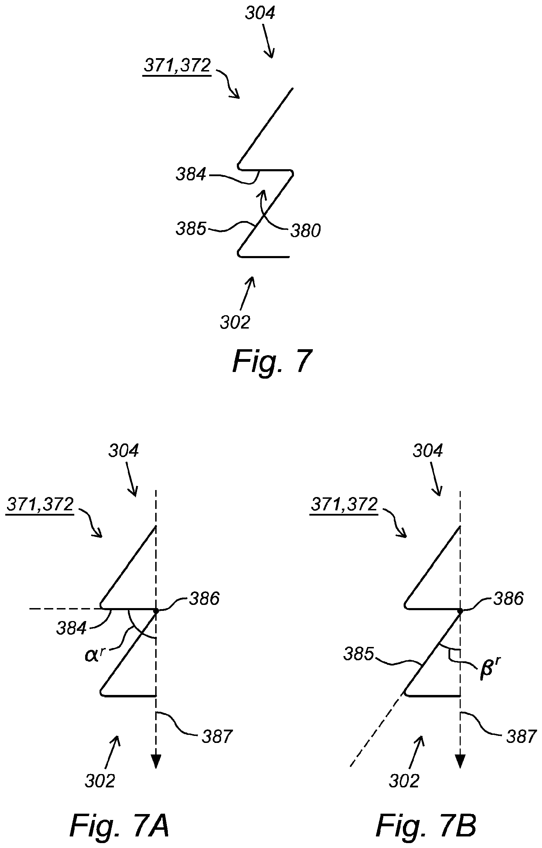

At least part of the recess PS and/or pawlPS may be planar and perpendicular to the direction movement of the first longitudinal member. This reduces slippage between the respective pushing surfaces. To further reduce slippage, the recess PS and/or pawlPS may be provided at least partially with a non-slip coating. Alternatively, at least part of the recess PS may be planar and inclined against the direction movement of the first longitudinal member **371.** In other words, and with reference to **FIG. 7A****,** the recess PS **384,** at the outermost edge **386,** may touch a fictive line **387** that is parallel to the direction of movement of the transmission element **373,** at an angle α^{r} measured from the distal side **302** of the angle. Angle α^{r} may be between 10° and 90°, preferably between 40° and 50 °.

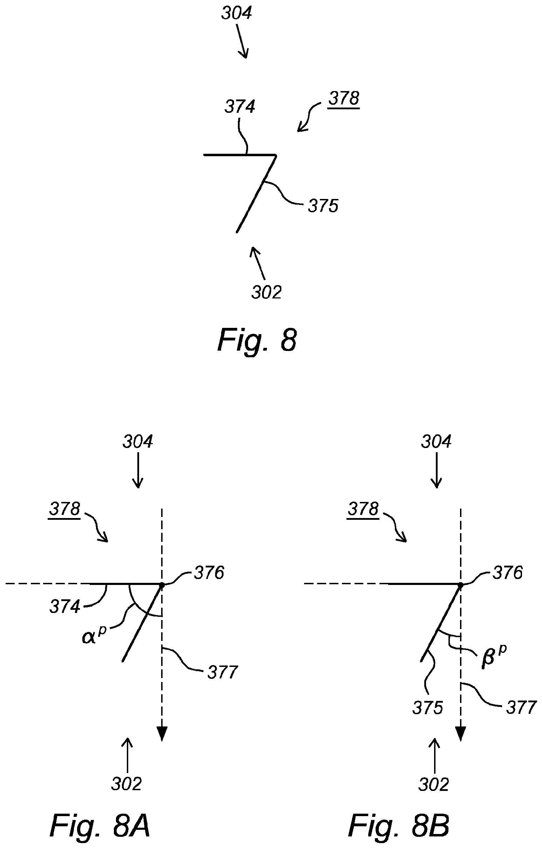

With reference to **FIG. 8A****,** the pawl PS **374,** at the outermost edge **376,** may touch a fictive line **377** that is parallel to the direction of movement of the transmission element **373,** at an angle α^{p} measured from the distal side **302** of the angle. Angle α^{p} may be between 10° and 90°, preferably between 40° and 50°.

The recess SS is adapted to slide past the pawl SS; force in the proximal direction applied by the first longitudinal member does not cause movement of the transmission element into a further index position, as the FLM recess-SS slides past the respective pawl SS. Force in the distal direction applied by the transmission element allows the transmission element to slide past the second longitudinal member, as the SLM recess-SS slides past the pawl SS.

The recess SS is configured to be in slidable relation with the pawl SS; this slidable configuration allows the transmission element to advance past the stationary second longitudinal member (during dispensing) and for the first longitudinal member to slide past the stationary transmission element during backward movement of first longitudinal member. At least part of the recess SS and/or pawl SS may be inclined to the direction movement of the first longitudinal member. This increases slippage between the respective sliding surfaces.

In other words, and with reference to **FIG. 7B****,** the recess SS **385,** at the outermost edge **386,** may touch a fictive line **387** that is parallel to the direction of movement of the transmission element **373,** at an angle β^{r} measured from the distal side **302** of the angle. Angle β^{r} may be between 10° and 80°, preferably between 40 ° and 50 °.

With reference to **FIG. 8B** the pawl SS **375,** at the outermost edge **376,** may touch a fictive line **377** that is parallel to the direction of movement of the transmission element **373,** at an angle β^{p} measured from the distal side **302** of the angle. Angle β^{p} may be between 10° and 80°, preferably between 40° and 50°. To further increase slippage, the recess SS **385** and/or pawl SS **375** may be provided at least partially with a slip coating.

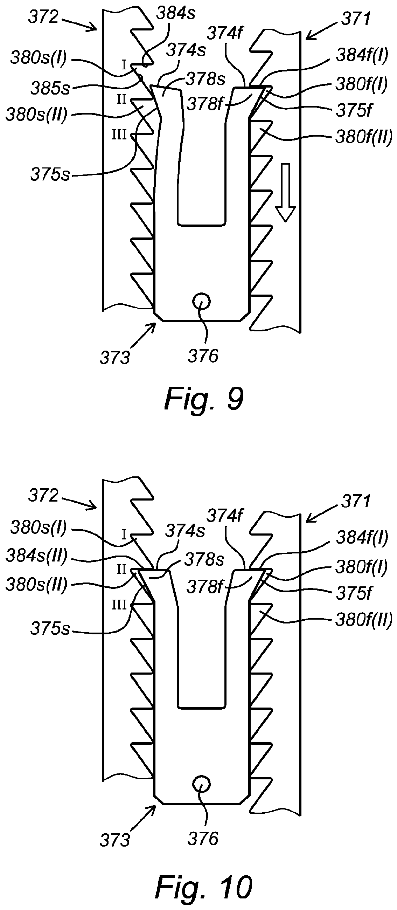

With reference to **FIG. 9** to **12****,** a further aspect of the invention provides a method for dispensing one solid support unit (111), from a SSU strip (1) with a dispenser (2) as defined herein, comprising the step of sliding the first longitudinal member (371) forward in the distal (203) direction, wherein the forward movement by the first longitudinal member (371) is transmitted via the transmission element (373) to the coupling (376), and the SSU strip advances by one solid support unit, thereby dispensing one solid support unit.

In certain embodiments of the method for dispensing one solid support unit, the method further comprises the step of moving the first longitudinal member (371) backwards in the proximal direction, thereby moving the first longitudinal member (371), wherein backward movement by the first longitudinal member (371) is not transmitted to the transmission element whose backward movement is prevented by engagement of the second extrusion with the serrated edge of the second longitudinal member (372).

In certain embodiments of the method for dispensing one solid support unit, the SSU strip moves only in the forward direction.

A further aspect provides a loading device configured for loading a receiving region of a holder as defined herein with a solid support unit as defined herein, the loading device comprising a longitudinal section for receiving the holder, a docking element for receiving a dispenser, and at least one loading gate configured for insertably receiving the distal (dispensing) end of a dispenser, wherein the loading gate connects said longitudinal section with the docking element. The loading gate may be a longitudinal void configured to insertably receive the distal dispensing end of the dispenser. The loading gate may have any suitable shape complementary with the shape of the dispenser, in particular complementary with the shape of the distal dispensing end of the dispenser. For example, the loading gate may be a longitudinal cylindrical void configured to insertably receive the distal dispensing end of the cylindrical dispenser.

The loading device facilitates loading a receiving region of a holder with a solid support unit, thereby reducing the time needed to load the holder and hence, reducing costs.

A related aspect provides a method for loading a receiving region of a holder as defined herein with a solid support unit as defined herein, comprising the steps of:
- providing a (partially or fully empty) holder as defined herein,
- providing a loading device as defined herein,
- inserting the (partially or fully empty) holder as defined herein in the loading device as defined herein,
- providing at least one SSU strip as defined herein, wherein the SSU strip is comprised in a dispenser configured to dispense one solid support unit at a time,
- inserting the distal (dispensing) end of the dispenser in one loading gate of the loading device,
- moving the first longitudinal member of the dispenser to advance one solid support unit from the SSU strip through the distal (dispensing) end in a receiving region of the holder, and
- turning the dispenser in order to detach the solid support unit from the SSU strip, thereby loading the receiving region of the holder with the solid support unit.

The dispenser may be turned by at least about 60° relative to the holder in order to break the solid support unit from the SSU strip. Preferably, the dispenser may be turned by at least about 90°, at least about 135°, or at least about 180°, relative to the holder.

In certain embodiment of the method for loading a receiving region of a holder as defined herein with a solid support unit as defined herein, the method may be performed manually. In certain embodiment of the method for loading a receiving region of a holder as defined herein with a solid support unit as defined herein, the method may be automated.

The present invention further provides a kit comprising at least one SSU strip as described herein and at least one dispenser as described herein. The SSU strip may be mounted inside the dispenser. The kit may further comprise the holder as described herein.

The present invention further provides a kit comprising at least one dispenser as defined herein, and at least one holder as defined herein.

Non-limiting examples of a SSU strip, a holder, a dispenser, a loading device, and a method for dispensing a solid support unit according to the present invention are illustrated in **FIG.s 1** to **14B****.**

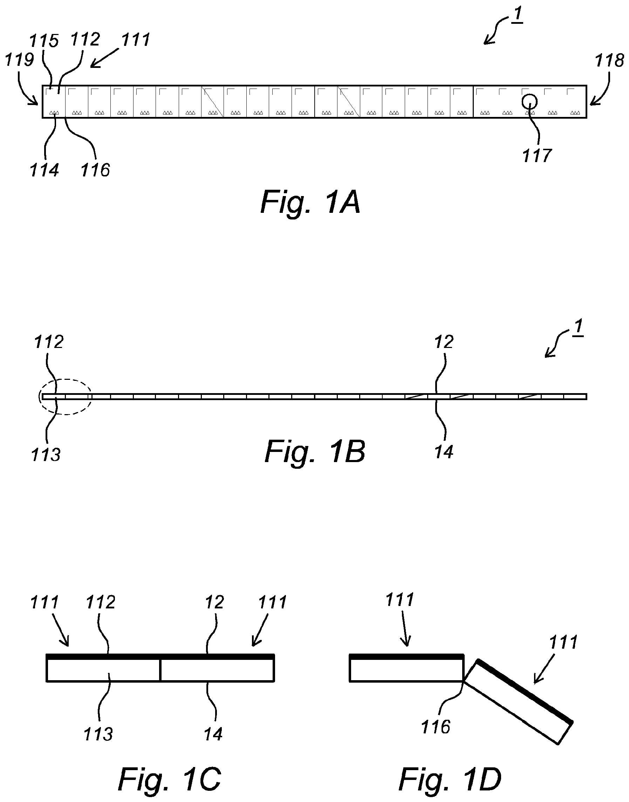

Referring to **FIG. 1A****,** a SSU strip 1 is shown comprising multiple, tandemly arranged solid support units (SSU) **111,** wherein each solid support unit **111** is identical within the SSU strip and each solid support unit **111** is detachable from the SSU strip **1.** The SSU strip **1** comprises at one end **118** a coupling element **117** for coupling to a dispenser configured to dispense one solid support unit **111** at a time. The other longitudinal end of the strip is the dispensing end **119.** Preferably, each solid support unit **111** comprises a membrane **112** (preferably an immunoassay membrane) provided on a backing layer (not shown). Each solid support unit **111** may comprise an identification element **114** and an orientation or positioning element **115.** The identification element allows the user to visually inspect or verify which type of solid support unit(s) has (have) been inserted into the holder. The orientation or positioning element allows an automatic reading or interpretation system to identify a reference position from which the different positions of the discrete test areas to be measured on the solid support unit can be extrapolated. Each solid support unit **111** is connected to its neighbouring solid support unit in part **116.**

Referring to **FIG. 1B****,** a side view of a SSU strip 1 is shown. The SSU strip **1** comprises a multitude of solid support units tandemly arranged, and connected to each other. Preferably, each solid support unit comprises a membrane **112** (preferably an immunoassay membrane) provided on a backing layer **113.** The SSU strip has an upper side **12** and an under side **14.**

Referring to **FIG. 1C****,** an enlarged side view of an end part of a SSU strip 1 is shown, wherein each solid support unit **111** comprises a membrane **112** provided on a backing layer **113.** An upper side **12** and under side **14** of each solid support unit **111** are indicated.

Referring to **FIG. 1D****,** an enlarged side view of an end part of a SSU strip 1 is shown, wherein the last solid support unit **111** is partially torn from the SSU strip and is connected to its neighbouring solid support unit in part **116** by the solid support backing **113.**

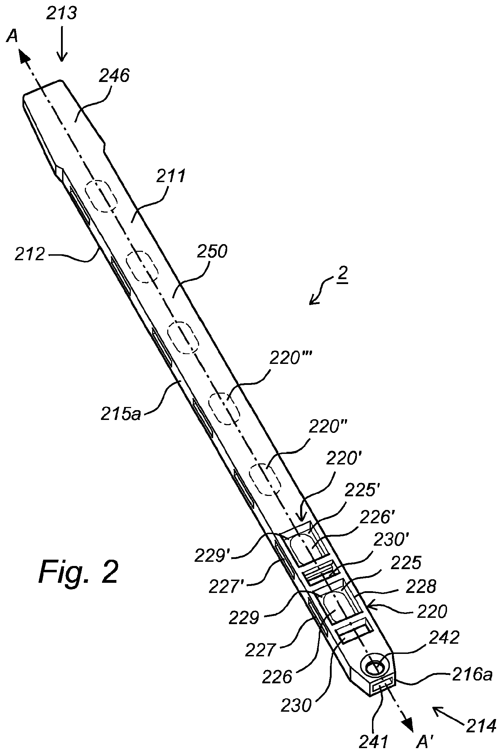

Referring to **FIG. 2****,** a holder **2** is shown, wherein the holder **2** comprises a longitudinal body **250** having a longitudinal axis (**A-A'**). The holder **2** has a front side **211,** a rear side **212,** a top end **213,** a base end **214.** A longitudinal side edge **215a** and a base end side edge **216a** are shown.

A plurality (pair) of receiving regions is shown that are compartments **220, 220'** spatially separated and arranged within the body **250.** Compartments **220, 220'** are shown towards the base end **214** of the holder **2.** Further compartments **220", 220'"** are shown only in outline. The pair of compartments **220, 220'** is separated by a spacing element **230'** that is a through passage between the front **211** and rear side **212** of the body. Each compartment comprises a front opening **228** that is an aperture in the front side **211** of the body, a rear wall **225** provided with a rear wall window **226,** and has an interior space **229.**

Each compartment is provided with a receptive opening that is a slot **227, 227'** in the longitudinal side edge **215a** of the body **250.** The slot **227, 227'** is configured to slidably receive a solid support unit for insertion into the compartment interior space **229.**

A sample chamber **242** is provided towards the base end **214** of the holder **2.** The sample chamber **242** is provided with a secondary opening **241** that connects the chamber void with the base end **214** side edge **216a.** Spacing element **230** spatially separates the sample chamber **242** from the end-most compartment **220.**

The holder **2** is provided with a handle **246** at the top end **213** of the body **250.**

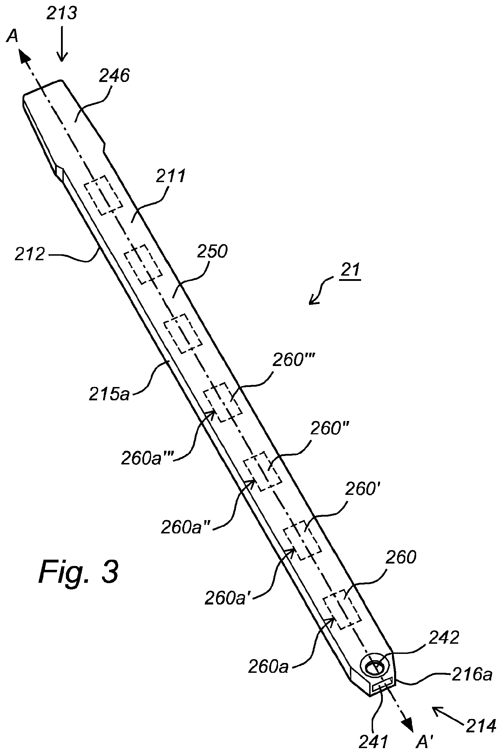

Referring to **FIG. 3****,** a holder **21** is shown, wherein the holder **21** comprises a longitudinal body **250** having a longitudinal axis (**A-A'**). The holder **21** has a front side **211,** a rear side **212,** a top end **213,** a base end **214.** A longitudinal side edge **215a** and a base end side edge **216a** are shown.

A plurality of receiving regions that are adhesive bonding surfaces **260, 260', 260", 260'"** is arranged along the longitudinal axis **A-A'** of the body **250.** Each adhesive bonding surface (*e.g.* **260**) is configured to receive a solid support unit and retain it by adhesion. Each adhesive bonding surface (*e.g.* **260**) is a delimited area arranged along the longitudinal axis **A-A'** of the body **250.** The delimitations **260a, 260a', 260a", 260a'"** provide an indication to the user about the position of each solid support unit. The delimitations (*e.g.* **260a**) are substantially complementary in size and shape with a solid support unit. Each adhesive bonding surface is spatially separated from any neighbouring adhesive bonding surface by a portion of the body. The adhesive bonding surfaces **260, 260', 260", 260'"** are arranged along the front side **211** of the body **250.** The delimited areas **260a, 260a', 260a", 260a'"** may comprise an adhesive, such as tape or glue coating, for fixing a solid support unit.

A sample chamber **242** is provided towards the base end **214** of the holder **21.** The sample chamber **242** is provided with a secondary opening **241** that connects the chamber void with the base end **214** side edge **216a.**

The holder **21** is provided with a handle **246** at the top end **213** of the body **250.**

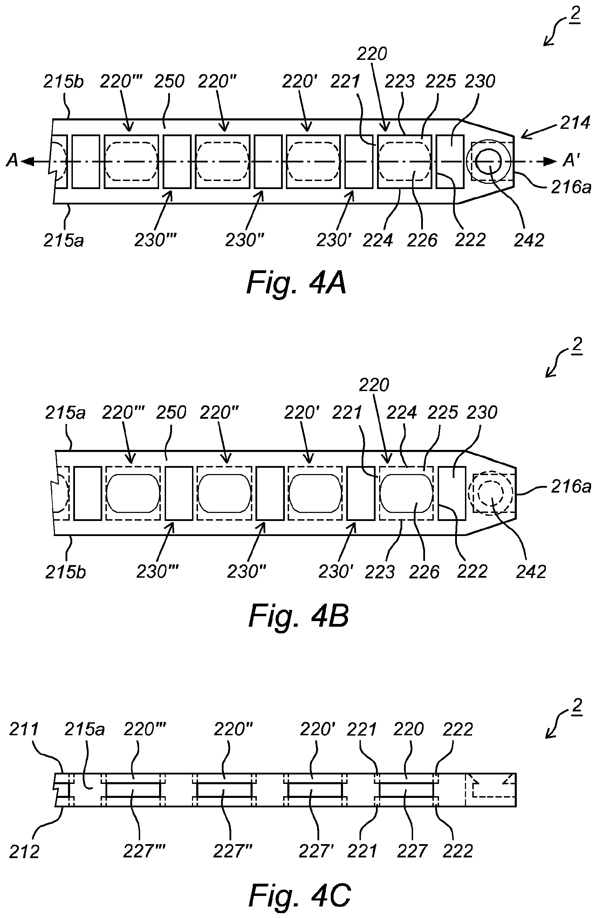

Referring to **FIG. 4A****,** a view of front side **211** of an enlarged part of a holder **2** of **FIG. 2** is shown. Four compartments are shown (**220, 220', 220", 220"'**). Each adjacent pair of compartments separated by a spacing element (**230', 230", 230"'**). The sample chamber **242** is separated from the end-most compartment **220** also by a spacing element **230.** Shown on the end-most compartment **220** is the rear wall **225,** and rear wall window **226,** and the side walls **221, 222, 223, 224** of the compartment **220.** The longitudinal side edges **215a, 215b,** and base end side edge **216a** are indicated.

Referring to **FIG. 4B****,** a view of rear side **212** of an enlarged part of a holder **2** is shown. The rear of four compartments are shown (**220, 220', 220", 220"'**), each adjacent pair of compartments separated by a spacing element (**230', 230", 230"'**). The solid base of sample chamber **242,** the longitudinal side edges **215a, 215b,** and base end side edge **216a** are indicated. Shown on the end-most compartment **220** is the rear wall **225,** and rear wall window **226,** and the side walls **221, 222, 223, 224** that enclose the compartment **220** shown through the rear-side **212** body **250.**

Referring to **FIG. 4C****,** a view of longitudinal side edge **215a** of an enlarged part of a holder **2** is shown. Four receptive openings are shown **227, 227', 227", 227"'** that are slots which connect the longitudinal side edge **215a** to the respective compartments (**220, 220', 220", 220"'**). Shown on the end-most compartment **220** are two side walls **221, 222** that partially enclose the compartment **220** and are behind the side edge **215a** body **250.**

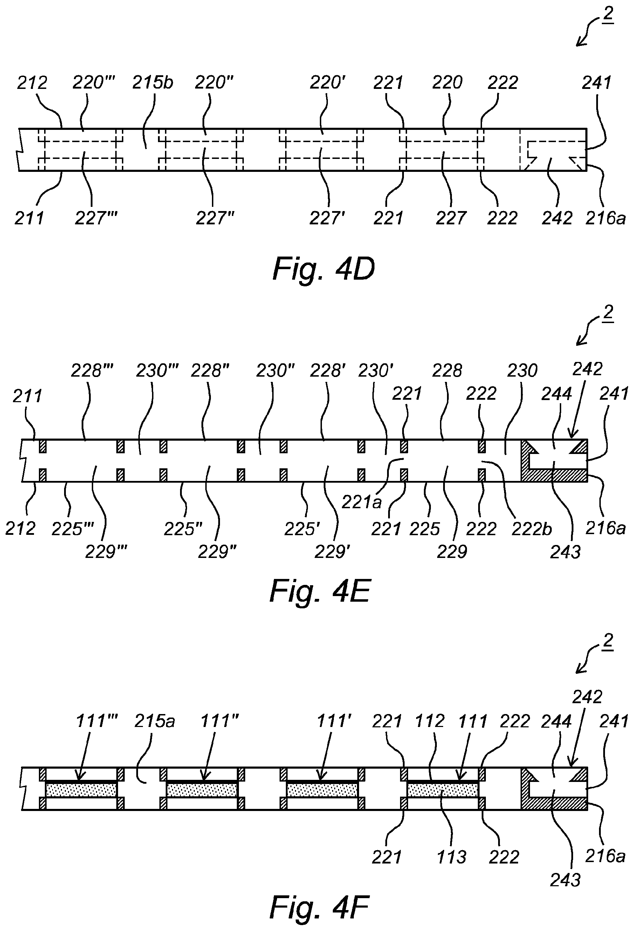

Referring to **FIG. 4D****,** a view of the other longitudinal side edge **215b** of an enlarged part of a holder **2** is shown. There are no receptive openings on this side edge **215b.** An indication of the four receptive openings **227, 227', 227", 227'"** on the other side edge **215a** is shown in outline. Shown on the end-most compartment **220** are two side walls **221, 222** that enclose the compartment **220** and are behind the side edge **215b** body **250.**

Referring to **FIG. 4E****,** a side view of a cross-section along the longitudinal axis A-A' of an enlarged part of a holder **2** is shown. Four compartments are shown (**220, 220', 220", 220"'**), each having a front opening (**228, 228', 228", 228"'**), a rear wall (**225, 225', 225", 225'"**), and an interior space (**229, 229', 229", 229"'**). Each adjacent pair of compartments separated by a spacing element (**230', 230", 230"'**), that is a through passage between the front **211** and rear side **212** of the body. The sample chamber **242** is separated from the end-most compartment **220** also by a spacing element **230.** The sample chamber **242** is provided with a principal opening **244** and a secondary opening **241** that connects the chamber void **243** with the base end **214** side edge (**216a**).

Shown on the end-most compartment **220** is the rear wall **225,** and rear wall window **226,** and two side walls **221, 222** that partially enclose the compartment **220.** The longitudinal side edges **215a, 215b,** and base end side edge **216a** are indicated. The side walls **221, 222** of the compartment each have an aperture **221a, 222a** that join opposite sides of said side wall together, i.e. connect the cavity of the spacing element **230** with the interior space of the compartment 229.

**FIG. 4F****,** is the same view depicted in **FIG. 3E****,** wherein each compartment **220** of the holder **2** comprises a solid support unit **111, 111', 111", 111"'.**

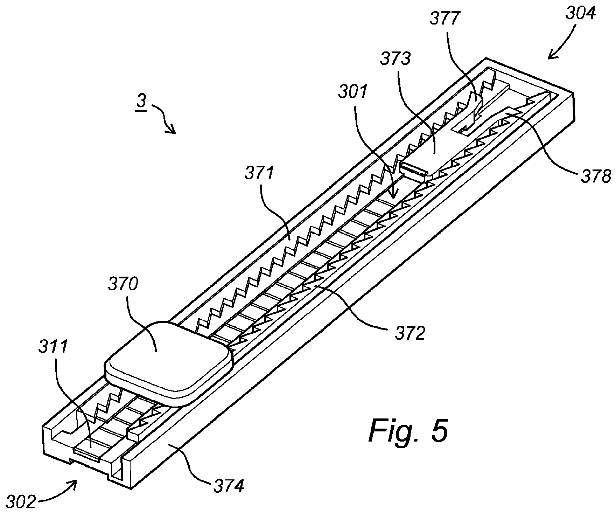

**FIG. 5** schematically represents a dispenser **3** according to an embodiment of the invention. The SSU strip **301** comprising multiple, tandemly arranged solid support units is comprised in the dispenser **3** configured to dispense one solid support unit **311** at a time. The dispenser **3** has, at one longitudinal end, a distal (dispensing) end **302,** through which the solid support unit **311** is advanced for dispensing, and at the other end, a proximal end **304.** The dispenser **3** comprises a first longitudinal member **371,** a second longitudinal member **372,** a transmission element **373,** and a longitudinal casing **374.** In fixed attachment to the first longitudinal member **371** is thumb pad **370,** for controlling a sliding movement of the first longitudinal member **371.** The first longitudinal member **371** and second longitudinal member **372** of the dispenser **3** each have an edge that is at least partly serrated. They are arranged parallel to each other in the casing **374.** The serrated edges are mutually adjacent.

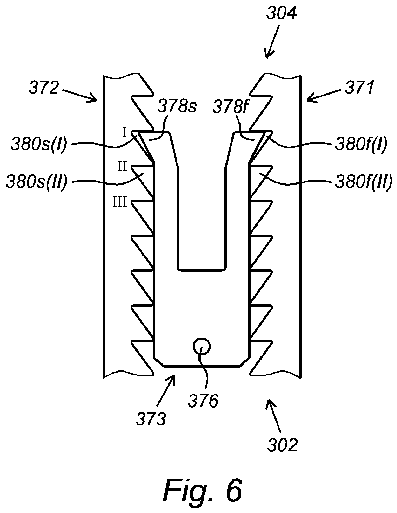

**FIG. 6** illustrates a transmission element **373** disposed in a gap between the first longitudinal member **371** and second longitudinal member **372.** It comprises a coupling **376** for dismountable or non-dismountable attachment to the SSU strip **301** (not shown). The transmission element **373** comprises a first **378f** and second **378s** pawl, which each engage with the serrated edge of the first **371** and second **372** longitudinal members respectively. The first pawl **378f** engages with a recess **380f(I)** of the serrated edge of the first longitudinal member **371;** shown is a neighbouring recess **380f(II).** The second pawl **378s** engages with a recess **380s(I)** of the serrated edge of the second longitudinal member **372;** shown is a neighbouring recess **380s(II).** The transmission element **373** is compliant, and applies a force between the opposing recesses.

**FIG. 7, 7a** and **7B** show a serrated edge in detail, more in particular a recess **380.** The recess of a longitudinal member comprises, at the proximal end **304** of the recess, a recess pushing surface (recess PS) **384,** and at the distal end **302,** a recess sliding surface (recess SS) **385.**

**FIG. 8, 8A** and **8B** shows a pawl **378** of a transmission element in detail. It comprises, at the proximal end **304** of the pawl, a pawl pushing surface (pawl PS) **374,** and at the distal end **302,** a recess sliding surface (recess SS) **375.**

**FIGs. 9 to 12** show a sequence of one-way forward movement by the transmission element **373** responsive to the forward and backward movements of the first longitudinal member **371.**

In **FIG. 9****,** forwards movement by the first longitudinal member **371** is transmitted to the coupling **376;** via a recess PS **384f(I)** - first pawl PS **374f** interaction. The transmission element **373** slides past the recess **380s(I)** of the second longitudinal member **372** owing to the sliding arrangement between recess SS **385s** of the second longitudinal member **372** and the second pawl SS **375s** of the transmission element. These surfaces are angled to slide past each other. Slippage is assisted by the compliance of the transmission element. **FIG. 10** shows the result of the movement; the transmission element **373** has advanced by one index (from I to II) relative to the second longitudinal member **372.**

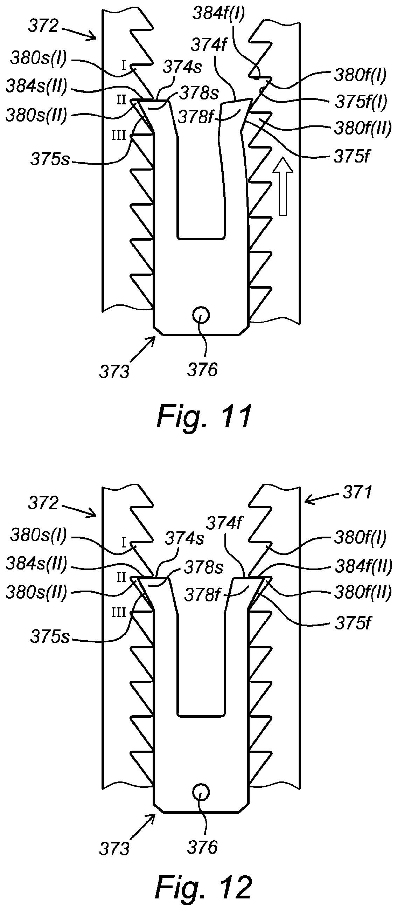

In **FIG. 11****,** backwards movement first longitudinal member **371** is not transmitted to the coupling **376.** The transmission element **373** slides past the recess **380f(I)** of the first longitudinal member **371** owing to the sliding arrangement between recess SS **385f(I)** of the first longitudinal member **371** and the first pawl SS **374f** of the transmission element. These surfaces are angled so that they slide past each another. Slippage is assisted by the compliance of the transmission element. The transmission element is further prevented from moving backwards by the interaction between the recess PS **384s (II)** and the second pawl PS **374s;** the recess PS **384s(II)** is an effective stop member.

**FIG. 12** shows the result of the movement; the transmission element **373** is still at index II of second longitudinal member **372,** but is now also at index II of the first longitudinal member **372.** Repeating the forward movement by the first longitudinal member **371** will advance the transmission into index position III.

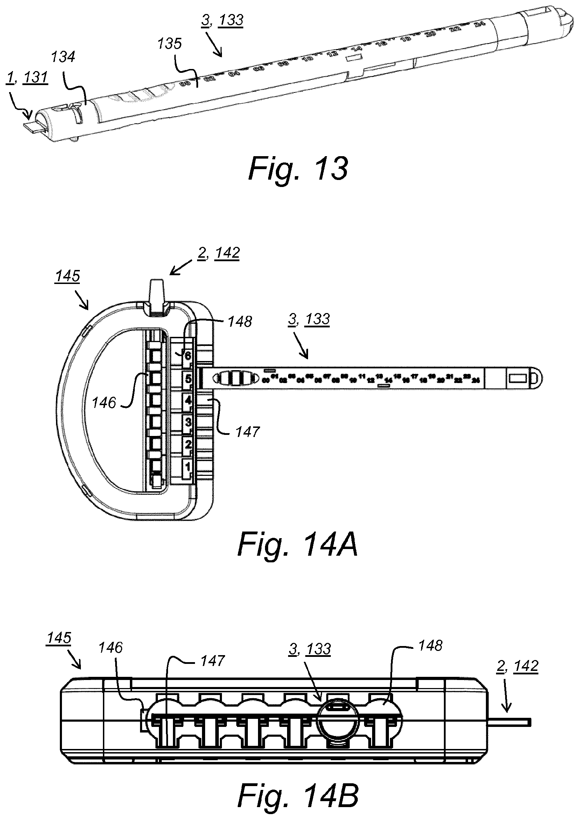

**FIG. 13** schematically represents a dispenser **3, 133** according to an embodiment of the invention. The SSU strip **1, 131** is comprised in the dispenser **3, 133** configured to dispense one solid support unit at a time. The dispenser **3, 133** has, at one longitudinal end, a distal (dispensing) end **134** through which the solid support units are advanced for dispensing one solid support unit at a time. The casing **135** of the dispenser **3, 133** is cylindrical.

**FIG. 14A** schematically represents an arrangement comprising a holder **2, 142** according to an embodiment of the present invention, a dispenser **3, 133** according to an embodiment of the invention, and a loading device **145** according to an embodiment of the present invention. **FIG. 14B** schematically represents a view of the arrangement of **FIG. 14A** along the longitudinal axis of the dispenser **3, 133.** The loading device **145** is configured for loading a receiving region of the holder **2, 142** with a solid support unit as defined herein. The loading device **145** comprises a longitudinal section **146** for receiving the holder **2, 142,** a docking element **147** for receiving a dispenser **3, 133,** and at least one loading gate **148** for insertably receiving the distal (dispensing) end of the dispenser **3, 133.** The loading device **145** comprises a longitudinal section **146** containing the holder **2, 142.** The loading device **145** further comprises six docking elements **147,** each for receiving a dispenser **3, 133.** The loading device **145** also comprises six loading gates **148** for insertably receiving the distal (dispensing) end of the dispenser **3, 133.** One docking element **147** in front of the loading gate **148** (indicated with number 5) contains the dispenser **3, 133.** The loading gate **148** (indicated with number 5) contains the distal end of the dispenser **3, 133.** The loading gate **148** connects said longitudinal section **146** with the docking element **147.** The loading gate **148** is a longitudinal cylindrical void configured to insertably receive the cylindrical distal dispensing end of the dispenser **3, 133.**

## Claims

1. A kit for preparing a custom-made test strip for detecting at least two analytes, the kit comprising at least one solid support unit (SSU) strip (1) and at least one holder (2, 21), wherein the SSU strip (1) comprises multiple, tandemly arranged solid support units (111), wherein each solid support unit (111) is identical within the SSU strip (1) and each solid support unit (111) is detachable from the SSU strip (1), and wherein the holder (2, 21) comprises a body (250) having a generally longitudinal shape, wherein said body (250) is provided with at least two receiving regions (220, 200', 260, 260'), wherein each receiving region (220, 220', 260, 260') is configured to receive a solid support unit (111), the receiving regions are arranged in a longitudinal direction along the body, the holder further comprises a sample chamber which is spatially separated from said receiving regions and dimensioned to hold a sample by means of capillarity, and wherein (a) each receiving region comprises a compartment (220, 220') in the body (250) of the holder (2) having a receptive opening for insertably receiving the solid support unit (111), and wherein the compartment is configured to mechanically secure the solid support unit, or (b) each receiving region comprises an adhesive bonding surface (260, 260') on the body (250) of the holder (2) configured to bond adhesively with the solid support unit (111).

2. The kit according to claim 1, wherein the kit comprises at least two different SSU strips.

3. The kit according to claim 1 or 2, wherein each solid support unit (111) comprises an immunoassay.

4. A solid support unit (SSU) strip (1) for preparing a custom-made test strip for detecting at least two analytes, the SSU strip (1) comprising multiple, tandemly arranged solid support units (111), wherein each solid support unit (111) is identical within the SSU strip (1) and each solid support unit (111) is detachable from the SSU strip (1), wherein each solid support unit (111) comprises an immunoassay.

5. The kit according to any one of claims 1 to 3, or the SSU strip according to claim 4, wherein the SSU strip is provided on a roll.

6. The kit according to any one of claims 1 to 3, or 5, or the SSU strip according to claim 4 or 5, wherein the SSU strip (1) comprises at one end a coupling element (117) for coupling to a dispenser (3) configured to dispense one solid support unit (111) at a time.

7. The kit according to any one of claims 1 to 3, 5, or 6, or the SSU strip according to any one of claims 4 to 6, wherein the SSU strip is comprised in a dispenser configured to dispense one solid support unit at a time.

8. A holder (2, 21) for preparing a custom-made test strip for detecting at least two analytes, the holder configured for holding at least two solid support units (111), the holder (2, 21) comprising a body (250) having a generally longitudinal shape, wherein said body (250) is provided with at least two receiving regions (220, 200', 260, 260'), wherein each receiving region (220, 220', 260, 260') is configured to receive a solid support unit (111), the receiving regions are arranged in a longitudinal direction along the body, the holder further comprises a sample chamber which is spatially separated from said receiving regions and dimensioned to hold a sample by means of capillarity, and wherein (a) each receiving region comprises a compartment (220, 220') in the body (250) of the holder (2) having a receptive opening for insertably receiving the solid support unit (111), and wherein the compartment is configured to mechanically secure the solid support unit, or (b) each receiving region comprises an adhesive bonding surface (260, 260') on the body (250) of the holder (2) configured to bond adhesively with the solid support unit (111).

9. The kit according to any of claims 1 to 3, 5 or 6, or the holder according to claim 8, wherein the receptive opening is a slot (227) in a longitudinal side wall (215a) of the body (250), which slot (227) is configured for slidably receiving the solid support unit and connects the outside of the body to an interior void space of the compartment (220, 220').

10. The kit according to any one of claims 1 to 3, 5 to 7, or 9, or the holder according to claim 8 or 9, wherein at least two receiving regions are each provided with a solid support unit (111).

11. Use of at least two different SSU strips and a holder, for preparing a custom-made test strip for detecting at least two analytes, wherein each SSU strip comprises multiple, tandemly arranged solid support units, wherein each solid support unit is identical within the SSU strip and each solid support unit is detachable from the SSU strip, and wherein the holder comprises a body having a generally longitudinal shape, wherein said body is provided with at least two receiving regions, wherein each receiving region is configured to receive a solid support unit and the holder further comprises a sample chamber which is spatially separated from said receiving regions and dimensioned to hold a sample by means of capillarity.

12. The use according to claim 11, wherein the SSU strips are defined as in any one of claims 4 to 7 and/or the holder is defined as in claim 8 or 9.

13. A method for preparing a custom-made test strip, comprising the steps of:
(a) providing at least two different solid support units (111),
(b) providing a holder (2, 21) comprising a body having a generally longitudinal shape, wherein said body is provided with at least two receiving regions, wherein each receiving region is configured to receive a solid support unit and the holder further comprises a sample chamber which is spatially separated from said receiving regions and dimensioned to hold a sample by means of capillarity, preferably a holder as defined in any one of claims 8 to 9, and
(c) loading at least two receiving regions (220, 220', 260, 260') of the holder (2, 21) each with a different solid support unit.

14. The method according to claim 13, wherein each solid support unit comprises an immunoassay.

15. The method according to claim 13 or 14 wherein the holder is as defined in any one of claims 8 to 9.

16. The method according to claim 13 or 15, for detecting at least two analytes in a biological sample, wherein the method further comprises the steps of:
(d) loading a biological sample in a sample chamber of said holder,
(e) inserting the loaded holder of step (d) in a container comprising at least one fluid, and
(f) detecting at least two analytes in the biological sample.

## Patentansprüche

1. Kit zur Herstellung eines benutzerspezifisch gefertigten Teststreifens zum Detektieren von mindestens zwei Analyten, wobei das Kit mindestens einen Festträgereinheits-(SSU)-Streifen (1) und mindestens einen Halter (2, 21) umfasst, wobei der SSU-Streifen (1) mehrere tandemartig angeordnete Festträgereinheiten (111) umfasst, wobei jede Festträgereinheit (111) innerhalb des SSU-Streifens (1) identisch ist und jede Festträgereinheit (111) von dem SSU-Streifen abgelöst werden kann, und wobei der Halter (2, 21) einen Körper (250) mit einer allgemein länglichen Form umfasst, wobei der Körper (250) mit mindestens zwei Aufnahmeregionen (220, 200', 260, 260') ausgestattet ist, wobei jede Aufnahmeregion (220, 220', 260, 260') ausgestaltet ist, um eine Festträgereinheit (111) aufzunehmen, wobei die Aufnahmeregionen in einer Längsrichtung entlang des Körpers angeordnet sind, der Halter ferner eine Probenkammer umfasst, die räumlich von den Aufnahmeregionen getrennt und so bemessen ist, dass sie mittels Kapillarwirkung eine Probe hält, und wobei (a) jede Aufnahmeregion ein Fach (220, 220') in dem Körper (250) des Halters (2) mit einer Aufnahmeöffnung zum einführbaren Aufnehmen der Festträgereinheit (111) umfasst, und wobei das Fach ausgestaltet ist, um die Festträgereinheit mechanisch zu sichern, oder (b) jede Aufnahmeregion eine Klebebindungsfläche (260, 260') auf dem Körper (250) des Halters (2) umfasst, die ausgestaltet ist, um klebend an die Festträgereinheit (111) zu binden.

2. Kit nach Anspruch 1, wobei das Kit mindestens zwei unterschiedliche SSU-Streifen umfasst.

3. Kit nach Anspruch 1 oder 2, wobei jede Festträgereinheit (111) einen Immunassay umfasst.

4. Festträgereinheits- (SSU)-Streifen (1) zur Herstellung von benutzerspezifisch gefertigten Teststreifen zum Detektieren von mindestens zwei Analyten, wobei der SSU-Streifen (1) mehrere, tandemartig angeordnete Festträgereinheiten (111) umfasst, wobei jede Festträgereinheit (111) innerhalb des SSU-Streifens (1) identisch ist und jede Festträgereinheit (111) von dem SSU-Streifen (1) abgelöst werden kann, wobei jede Festträgereinheit (111) einen Immunassay umfasst.

5. Kit nach einem der Ansprüche 1 bis 3 oder SSU-Streifen nach Anspruch 4, wobei der SSU-Streifen auf einer Rolle bereitgestellt wird.

6. Kit nach einem der Ansprüche 1 bis 3 oder 5, oder SSU-Streifen nach Anspruch 4 oder 5, wobei der SSU-Streifen (1) an einem Ende ein Kopplungselement (117) zum Koppeln mit einem Spender (3) umfasst, der ausgestaltet ist, um eine Festträgereinheit (111) zur Zeit abzugeben.

7. Kit nach einem der Ansprüche 1 bis 3, 5 oder 6, oder SSU-Streifen nach einem der Ansprüche 4 bis 6, wobei der SSU-Streifen in einem Spender enthalten ist, der ausgestaltet ist, um eine Festträgereinheit zur Zeit abzugeben.

8. Halter (2, 21) zum Herstellen eines benutzerspezifisch gefertigten Teststreifens zum Detektieren von mindestens zwei Analyten, wobei der Halter ausgestaltet ist, um mindestens zwei Festträgereinheiten (111) zu halten, wobei der Halter (2, 21) einen Körper (250) mit einer allgemein länglichen Form umfasst, wobei der Körper (250) mit mindestens zwei Aufnahmeregionen (220, 200', 260, 260') ausgestattet ist, wobei jede Aufnahmeregion (220, 220', 260, 260') ausgestaltet ist, um eine Festträgereinheit (111) aufzunehmen, wobei die Aufnahmeregionen in einer Längsrichtung entlang des Körpers angeordnet sind, der Halter ferner eine Probenkammer umfasst, die räumlich von den Aufnahmeregionen getrennt und so bemessen ist, dass sie eine Probe mittels Kapillarwirkung hält, und wobei (a) jede Aufnahmeregion ein Fach (220, 220') in dem Körper (250) des Halters (2) mit einer Aufnahmeöffnung umfasst, um die Festträgereinheit (111) durch Einsetzen aufzunehmen, und wobei das Fach ausgestaltet ist, um die Festträgereinheit mechanisch zu sichern, oder (b) jede Aufnahmeregion eine Klebebindungsfläche (260, 260') auf dem Körper (250) des Halters (2) umfasst, die ausgestaltet ist, um klebend an die Festträgereinheit (111) zu binden.

9. Kit nach einem der Ansprüche 1 bis 3, 5 oder 6, oder Halter nach Anspruch 8, wobei die Aufnahmeöffnung ein Schlitz (227) in einer Längsseitenwand (215a) des Körpers (250) ist, wobei der Schlitz (227) zum gleitenden Aufnehmen der Festträgereinheit ausgestaltet ist und die Außenseite des Körpers mit einem inneren Hohlraum des Fachs (220, 220') verbindet.

10. Kit nach einem der Ansprüche 1 bis 3, 5 bis 7 oder 9 oder Halter nach Anspruch 8 oder 9, wobei mindestens zwei Aufnahmeregionen jeweils mit einer Festträgereinheit (111) ausgestattet sind.

11. Verwendung von mindestens zwei unterschiedlichen SSU-Streifen und einem Halter zum Herstellen eines benutzerspezifisch gefertigten Teststreifens zum Detektieren von mindestens zwei Analyten, wobei jeder SSU-Streifen mehrere tandemartig angeordnete Festträgereinheiten umfasst, wobei jede Festträgereinheit innerhalb des SSU-Streifens identisch ist und jede Festträgereinheit von dem SSU-Streifen abgelöst werden kann, und wobei der Halter einen Körper mit einer allgemein länglichen Form umfasst, wobei der Körper mit mindestens zwei Aufnahmeregionen ausgestattet ist, wobei jede Aufnahmeregion ausgestaltet ist, um eine Festträgereinheit aufzunehmen, und der Halter ferner eine Probenkammer umfasst, die räumlich von den Aufnahmeregionen getrennt ist und so bemessen ist, dass sie eine Probe mittels Kapillarwirkung hält.

12. Verwendung nach Anspruch 11, wobei die SSU-Streifen wie in einem der Ansprüche 4 bis 7 definiert sind und/oder der Halter wie in Anspruch 8 oder 9 definiert ist.

13. Verfahren zum Herstellen eines benutzerspezifisch gefertigten Teststreifens, umfassend die Schritte:
(a) Bereitstellen von mindestens zwei verschiedenen Festträgereinheiten (111),
(b) Bereitstellen eines Halters (2, 21), der einen Körper mit allgemein länglicher Form umfasst, wobei der Körper mit mindestens zwei Aufnahmeregionen ausgestattet ist, wobei jede Aufnahmeregion so ausgestaltet ist, dass sie eine Festträgereinheit aufnimmt, und der Halter ferner eine Probenkammer umfasst, die räumlich von den Aufnahmeregionen getrennt und so bemessen ist, dass sie eine Probe mittels Kapillarwirkung hält, vorzugsweise eines Halters wie in einem der Ansprüche 8 bis 9 definiert, und
(c) Beladen von mindestens zwei Empfangsregionen (220, 220', 260, 260') des Halters (2, 21) jeweils mit einer unterschiedlichen Festträgereinheit.

14. Verfahren nach Anspruch 13, wobei jede Festträgereinheit einen Immunassay umfasst.

15. Verfahren nach Anspruch 13 oder 14, wobei der Halter wie in einem der Ansprüche 8 bis 9 definiert ist.

16. Verfahren nach Anspruch 13 oder 15 zum Detektieren von mindestens zwei Analyten in einer biologischen Probe, wobei das Verfahren ferner die Schritte umfasst:
(d) Laden einer biologischen Probe in eine Probenkammer des Halters,
(e) Einsetzen des beladenen Halters aus Schritt (d) in einen Behälter, der mindestens ein Fluid umfasst, und
(f) Detektieren von mindestens zwei Analyten in der biologischen Probe.

## Revendications

1. Kit pour la production d'une bandelette d'analyse personnalisée pour la détection d'au moins deux analytes, le kit comprenant au moins une bandelette d'unités de support solides (USS) (1) et au moins un support (2, 21), la bandelette d'USS (1) comprenant des unités de support solides multiples (111) disposées en tandem, chaque unité de support solide (111) étant identique dans la bandelette d'USS (1) et chaque unité de support solide (111) étant détachable de la bandelette d'USS (1), et le support (2, 21) comprenant un corps (250) ayant une forme généralement longitudinale, ledit corps (250) étant pourvu d'au moins deux régions de réception (220, 200', 260, 260'), chaque région de réception (220, 220', 260, 260') étant configurée pour recevoir une unité de support solide (111), les régions de réception étant disposées en une direction longitudinale le long du corps, le support comprenant en outre une chambre à échantillon qui est séparée spatialement desdites régions de réception et dimensionnée pour maintenir un échantillon par capillarité, et (a) chaque région de réception comprenant un compartiment (220, 220') dans le corps (250) du support (2), comportant une ouverture de réception destinée à recevoir par insertion l'unité de support solide (111), et le compartiment étant conçu pour maintenir mécaniquement l'unité de support solide, ou (b) chaque région de réception comprenant une surface de fixation adhésive (260, 260') sur le corps (250) du support (2), configurée pour se fixer par adhérence à l'unité de support solide (111).

2. Kit selon la revendication 1, le kit comprenant au moins deux bandelettes d'USS différentes.

3. Kit selon la revendication 1 ou 2, dans lequel chaque unité de support solide (111) comprend un dosage immunologique.

4. Bandelette d'unités de support solides (USS) (1) pour la production d'une bandelette d'analyse personnalisée pour la détection d'au moins deux analytes, la bandelette d'USS (1) comprenant de multiples unités de support solide (111) disposées en tandem, chaque unité de support solide (111) étant identique dans la bandelette d'USS (1) et chaque unité de support solide (111) étant détachable de la bandelette d'USS (1), chaque unité de support solide (111) comprenant un dosage immunologique.

5. Kit selon l'une quelconque des revendications 1 à 3, ou bandelette d'USS selon la revendication 4, dans lesquels la bandelette d'USS est fournie sur un rouleau.

6. Kit selon la revendication 1 à 3, ou 5, ou bandelette d'USS selon la revendication 4 ou 5, dans lesquels la bandelette d'USS (1) comprend à une extrémité un élément d'appariement (117) pour l'appariement à un distributeur (3) conçu pour distribuer une unité de support solide (111) à la fois.

7. Kit selon l'une quelconque des revendications 1 à 3, 5, ou 6, ou bandelette d'USS selon l'une quelconque des revendications 4 à 6, dans lesquels la bandelette d'USS est insérée dans un distributeur conçu pour distribuer une unité de support solide à la fois.

8. Support (2, 21) pour la production d'une bandelette d'analyse personnalisée pour la détection d'au moins deux analytes, le support étant configuré pour le maintien d'au moins deux unités de support solides (111), le support (2, 21) comprenant un corps (250) ayant une forme généralement longitudinale, ledit corps (250) étant pourvu d'au moins deux régions de réception (220, 200', 260, 260'), chaque région de réception (220, 220', 260, 260') étant configurée pour recevoir une unité de support solide (111) les régions de réception étant disposées en une direction longitudinale le long du corps, le support comprenant en outre une chambre à échantillon qui est séparée spatialement desdites régions de réception et dimensionnée pour maintenir un échantillon par capillarité, et (a) chaque région de réception comprenant un compartiment (220, 220') dans le corps (250) du support (2), comportant une ouverture de réception destinée à recevoir par insertion l'unité de support solide (111), et le compartiment étant conçu pour maintenir mécaniquement l'unité de support solide, ou (b) chaque région de réception comprenant une surface de fixation adhésive (260, 260') sur le corps (250) du support (2), configurée pour se fixer par adhérence à l'unité de support solide (111).

9. Kit selon l'une quelconque des revendications 1 à 3, 5 ou 6, ou support selon la revendication 8, dans lesquels l'ouverture de réception est une fente (227) dans une paroi latérale longitudinale (215a) du corps (250), laquelle fente (227) est configurée pour recevoir par coulissement l'unité de support solide et met en communication l'extérieur du corps avec un espace intérieur vide du compartiment (220, 220').

10. Kit selon l'une quelconque des revendications 1 à 3, 5 à 7, ou 9, ou support selon la revendication 8 ou 9, dans lesquels au moins deux régions de réception sont munies chacune d'une unité de support solide (111).

11. Utilisation d'au moins deux bandelettes d'USS différentes et d'un support pour la production d'une bandelette d'analyse personnalisée pour la détection d'au moins deux analytes chaque bandelette d'USS comprenant de multiples unités de support solides disposées en tandem, chaque unité de support solide étant identique dans la bandelette d'USS et chaque unité de support solide étant détachable de la bandelette d'USS, et le support comprenant un corps ayant une forme généralement longitudinale, ledit corps étant pourvu d'au moins deux régions de réception, chaque région de réception étant configurée pour recevoir une unité de support solide et le support comprenant en outre une chambre à échantillon qui est séparée spatialement desdites régions de réception et dimensionnée pour maintenir un échantillon par capillarité.

12. Utilisation selon la revendication 11, pour laquelle les bandelettes d'USS sont telles que définies dans l'une quelconque des revendications 4 à 7 et/ou le support est tel que défini dans la revendication 8 ou 9.

13. Procédé pour la production d'une bandelette d'analyse personnalisée, comprenant les étapes consistant à:
(a) fournir au moins deux unités de support solides différentes (111),
(b) fournir un support (2, 21) comprenant un corps ayant une forme généralement longitudinale, ledit corps étant pourvu d'au moins deux régions de réception, chaque région de réception étant configurée pour recevoir une unité de support solide et le support comprenant en outre une chambre à échantillon qui est séparée spatialement desdites régions de réception et dimensionnée pour maintenir un échantillon par capillarité, de préférence un support tel que défini dans l'une quelconque des revendications 8 et 9, et
(c) charger au moins deux régions de réception (220, 220', 260, 260') du support (2, 21) chacune avec une unité de support solide différente.

14. Procédé selon la revendication 13, dans lequel chaque unité de support solide comprend un dosage immunologique.

15. Procédé selon la revendication 13 ou 14, dans lequel le support est tel que défini dans l'une quelconque des revendications 8 et 9.

16. Procédé selon la revendication 13 ou 15, pour la détection d'au moins deux analytes dans un échantillon biologique, le procédé comprenant en outre les étapes consistant à:
(d) charger un échantillon biologique dans une chambre à échantillon dudit support,
(e) insérer le support chargé de l'étape (d) dans un récipient comprenant au moins un fluide, et
(f) détecter au moins deux analytes dans l'échantillon biologique.
